(19) Europäisches Patentamt
European Patent Office
Office européen des brevets

(11) Publication number: **0 655 498 A1**

# (12) EUROPEAN PATENT APPLICATION

(21) Application number: **94250286.5**

(22) Date of filing: **29.11.94**

(51) Int. Cl.[6]: **C12N 15/12**, C12N 15/70, C07K 14/47, C07K 1/14, C12Q 1/68

(30) Priority: **30.11.93 US 160087**

(43) Date of publication of application: **31.05.95 Bulletin 95/22**

(84) Designated Contracting States: **AT BE CH DE DK ES FR GB GR IE IT LI LU MC NL PT SE**

(71) Applicant: **Oklahoma Medical Research Foundation**
**825 N.E. 13th Street**
**Oklahoma City**
**Oklahoma 73104 (US)**

(72) Inventor: **Conaway, Ronald Charles**
**10801 East Apple Valley Rd., Oklahoma City**
**Oklahoma 73151 (US)**
Inventor: **Conaway, Joan Weliky**
**10801 East Apple Valley Rd., Oklahoma City**
**Oklahoma 73151 (US)**
Inventor: **Bradsher, John N.,**
**420 E. 70th Street, Apt. 9E,**
**New York, N.Y. 10021 (US)**

(74) Representative: **UEXKÜLL & STOLBERG**
**Patentanwälte**
**Beselerstrasse 4**
**D-22607 Hamburg (DE)**

## (54) RNA polymerase II transcription factor.

(57) RNA polymerase transcription factor S111 has been isolated and characterised which increases the rate of transcription elongation by RNA polymerase II.

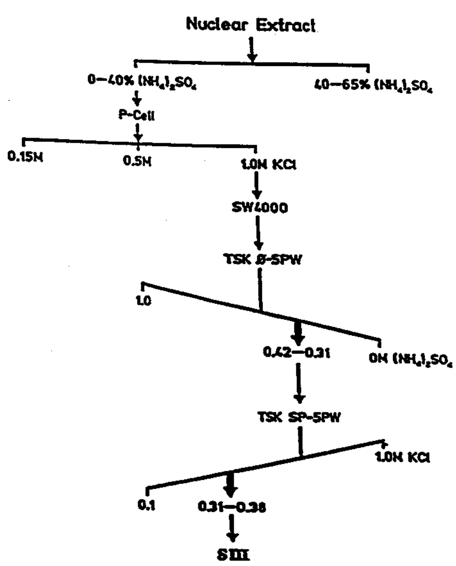


Fig. 1

EP 0 655 498 A1

## RELATED APPLICATION

This is a continuation-in-part of copending United States application Serial No. 08/160,087 filed November 30, 1993.

## TECHNICAL FIELD

This invention relates to the field of RNA polymerase transcription factors.

## GOVERNMENT RIGHTS

The invention described herein was made in the course of work under a grant or award from The National Institutes of Health, and the Government may have certain rights therein.

## BACKGROUND OF THE INVENTION

Transcription is the first stage in gene expression and the principal step at which it is controlled. It involves synthesis of an RNA chain representing the coding strand of a DNA duplex. Transcription takes place by the usual process of complementary base pairing catalyzed by the enzyme RNA polymerase. This reaction can be divided into three stages: initiation; elongation; and termination.

In eucaryotic systems, RNA polymerase associates with several other enzymes and factors at a sequence of DNA which defines a promoter. Accurate initiation requires a number of initiation factors and is an important point at which transcription is controlled.

Elongation describes the phase during which the RNA polymerase moves along the DNA and extends the growing RNA chain. As the RNA polymerase moves, it unwinds the DNA helix to expose a new segment of the template in single-stranded form. Nucleotides are covalently added to the 3' end of the growing RNA chain forming an RNA-DNA hybrid in the unwound region. Additionally, the RNA that was made previously is displaced from the DNA template strand, which pairs with its original partner to reform the duplex helix. Thus, elongation involves the disruption of DNA structure to form a transiently unwound region that exists as a hybrid RNA-DNA duplex and a displaced single strand of DNA.

Termination involves recognition by the RNA polymerase of the point at which no further bases should be added to the chain, followed by dissociation of RNA polymerase from both the DNA template and the newly synthesized RNA chain.

Messenger RNA synthesis is a complex biochemical process requiring the action of multiple transcription factors, including initiation and elongation factors, that control the activity of the RNA polymerase at both the initiation and elongation stages of transcription. Several of these factors are known to be essential for initiation and are referred to as factors d, e, a, g, and b from *Saccharomyces cerevisiae,* $\tau$, $\alpha$, $\beta\gamma$, $\delta$ and $\epsilon$ from rat liver, and TFIID, TFIIB, RAP30/74 or TFIIF, BTF2 or TFIIH, and TFIIE from human cells.

In addition to these factors, other proteins have been shown to stimulate either the initiation or elongation stages of transcription by RNA polymerase II. One such factor, designated TFIIA, has been purified from both *Saccharomyces cerevisiae* and mammalian cells and appears to promote assembly of the preinitiation complex. Although TFIIA is not essential for initiation, several lines of evidence suggest that it functions to increase the number of productive preinitiation complexes formed at a variety of promoters *in vitro.*

Although considerable progress has recently been achieved identifying and characterizing transcription factors that support a basal level of transcription by RNA polymerase II, significantly less information is available on transcription factors that control the efficiency of transcription initiation or RNA chain elongation. Such activities play an important role in regulating gene expression.

Only two transcription factors that influence RNA chain elongation have been identified and characterized with a high degree of certainty. The general initiation factor TFIIF (RAP 30/74) from *Drosophila* and human cells has been shown to stimulate the rate of RNA chain elongation and to promote read-through by RNA polymerase at a variety of pause sites. Transcription factor SII has been shown to promote RNA polymerase read-through at intrinsic pause sites in a human histone gene, in the adenovirus genome, and at several other sites.

A transcription factor designated YES was recently purified to apparent homogeneity from *Saccharomyces cerevisiae*. YES is composed of a single 115 kDa polypeptide and appears to stimulate the rate of RNA chain elongation by RNA polymerase on synthetic oligo(dC)-tailed DNA templates, although its existence is now in question. Additionally, a transcription factor designated TFIIX has been described.

However, it has not yet been purified; thus, it is not yet clear how it is related to other better characterized elongation factors.

Many of the initiation factors and one or more elongation factors (e.g., TFIIF) are commercially available from most suppliers of biological enzymes (e.g., Upstate Biotechnology, Promega Corp., and Santa Cruz Biotechnology, Inc.). The ability of RNA polymerase to form an active complex *in vitro* capable of specifically initiating and efficiently elongating RNA is vital to the development of genetically engineered industrial systems for the production of recombinant products. Furthermore, other as yet unknown elongation factors hold the key to many cellular functions. Thus, the ability to manipulate the transcription of genes is necessary to overcome many hurdles in genetic engineering and will be required to cure several human diseases.

One such area which relies upon elucidation of genetic mechanisms is the effort to understand the etiology and treatment of diseases caused by viruses. For example, one of the most intensive efforts in recent years has focused on the HIV virus which causes Acquired Immune Deficiency Syndrome (AIDS). It has been reported that the HIV encoded Tat protein recruits unknown cofactors to the HIV-1 LTR TAR element (Madore, et al., *J Virology* 67: 3703 (1993)) thereby inducing activation of viral gene expression. There is reason to believe the unknown co-factors are likely to be elongation factors, and thus, further identification of elongation factors are important in elucidating genetic means for treating AIDS. In addition, as the HIV Tat protein is itself believed to be an elongation factor, cellular elongation factors will be important components of assay systems designed to identify pharmacologic agents that interfere with the activity of the HIV Tat protein. (Rosen, C.A., *AIDS Research and Human Retroviruses* 8: 175-181 (1992)).

Elongation factors are also believed to be involved with the repair of actively transcribed genes. Transcribed genes are repaired on a priority basis because the resulting translation product would likely be either a mutated full-length product or a truncated product (due to premature termination of transcription). Elongation factors would be a likely signal for repair systems such as ERCC 3 because they are one of the few enzymes which are associated with DNA only when mRNA is actually being elongated. Elucidation of this signal could result in a treatment for certain repair deficiency diseases such as xeroderma pigmentosum and Cockaynes syndrome.

Elongation factors are also likely to be involved in the regulation of a number of cellular genes including the proto oncogene C-myc and genes involved in the cellular stress response.

In brief, the presence or absence of these elongation factors controls the rate of transcription of many genes and their absence can even prevent continued transcription. They are certainly involved in the coordination of transcription with other cellular functions. However, despite the advances made to date in the field of genetic engineering, there is a continuing need for novel factors which affect the rate and efficiency of transcription.

## SUMMARY OF THE INVENTION

A novel RNA polymerase transcription factor is provided. Increasing the available amount of this protein in transcription systems significantly increases the rate of transcription.

The present invention further comprises a method of isolating the SIII protein from eukarotic cells. For example rat brain and rat liver cells can be used as sources of this novel transcription factor.

The present invention is also directed toward the cDNAs encoding the rat ~15, ~18 and ~110 kDa subunits of SIII.

The present invention is also directed toward the recombinantly produced SIII protein as well as the vectors and transformation systems which make that possible.

The present invention also comprises the cDNA encoding the human ~15 kDa subunit and cDNAs encoding portions of the human ~18 and ~110 kDa subunits of SIII.

These and other features, aspects and advantages of the present invention will become better understood with reference to the following description and appended claims.

## BRIEF DESCRIPTION OF THE DRAWINGS

Fig. 1 is a preferred purification scheme of RNA polymerase transcription factor SIII from rat liver, where P-cell represents phosphocellulose and $\phi$ represents a phenyl group.

Fig. 2 is a graph depicting the results of runoff transcription assays which illustrate SIII stimulating transcription in a dose-dependent manner.

Fig. 3 is a graph depicting the results of recombinantly produced SIII transcriptional activity. In some instances certain subunits were omitted.

DETAILED DESCRIPTION

The following description provides details of the manner in which the embodiments of the present invention may be made and used in order to achieve the separation, purification and characterization of an RNA polymerase elongation factor which was not previously known. Also included is a description for the recombinant production of the elongation factor. These descriptions, while exemplary of the present invention, are not to be construed as specifically limiting the invention, and such variations which would be within the purview of one skilled in this art are to be considered to fall within the scope of this invention.

Definitions:

The abbreviations used are: AMP-PNP, adenyl-5'-yl imidodiphosphate; PMSF, phenylmethylsulfonyl fluoride; TFA, trifluoroacetic acid; HPLC, high pressure liquid chromatography; DTT, dithiothreitol; HEPES, N-[2-hydroxyethyl]piperazine-N'-[2-ethanesulfonic acid]; SDS, sodium dodecyl sulfate; EDTA, ethylenediaminetetraacetic acid; AdML, adenovirus 2 major late; USF, upstream stimulatory factor; and MLTF, major late transcription factor. "~" means approximately. Whenever a nucleic acid is referred to, either directly or inferentially, it may be meant to include the nucleic acid in both the sense and anti-sense orientations.

A new RNA polymerase transcription factor has now been isolated. This transcription factor can be used to increase the rate of transcription by stimulating synthesis by RNA polymerase. Several lines of evidence demonstrate that this transcription factor, designated SIII, is a heterotrimeric protein composed of approximately 110(p110), 18(p18), and 15(p15) kDa subunits. These are: (i) polypeptides of approximately 110, 18, and 15 kDa compose at least about 90% of the protein in the most highly purified preparations of SIII; (ii) SIII activity co-chromatographs with the approximately 110, 18, and 15 kDa polypeptides during both ion-exchange and hydrophobic interaction HPLC; (iii) SIII activity can be reconstituted by recombining the approximately 110, 18, and 15 kDa polypeptides isolated by reverse phase HPLC; and (iv) based on the results of gel filtration and sedimentation experiments, SIII exhibits a native molecular mass of about 140 kDa, consistent with the idea that it contains a single copy of each of the three polypeptides.

SIII is structurally distinct from the TATA factor ($\tau$/TFIID), its DNA binding subunit (the TATA Binding Protein or TBP), and general initiation factors $\alpha$(TFIIB), $\beta\gamma$(TFIIF), $\delta$ (BTF2/TFIIH), and $\epsilon$ (TFIIE) . Nor will it replace any of these factors in reconstitution of promoter-specific initiation. In addition, its polypeptide composition differs from that of TFIIA which stimulates transcription initiation by RNA polymerase. SIII also appears to be distinct from elongation factors designated SII and TFIIX. Mammalian SII is composed of a single, 38 kDa polypeptide, and thus differs structurally from SIII. TFIIX, on the other hand, has thus far been defined only as an activity present in a chromatographic fraction from HeLa cells. However, while both SIII and the TFIIX activity bind tightly to phosphocellulose and are eluted between 0.5 and 1.0 M KCl, SIII flows through DEAE cellulose at 0.1 M KCl (data not shown), while TFIIX binds this resin and is eluted between 0.1 and 0.5 M KCl.

The following examples describe experiments investigating the functional properties of SIII. These indicate that SIII stimulates promoter specific transcription by increasing the rate of RNA chain elongation by RNA polymerase. The following observations argue that SIII exerts its activity directly on the elongation complex: (i) in pulse chase experiments, SIII does not need to be present during preinitiation complex formation or transcription initiation in order to stimulate transcription; and (ii) SIII stimulates the rate of RNA chain elongation during transcription of double stranded oligo dC-tailed templates. In this respect, the activity of SIII resembles that of the well-characterized transcription elongation factor SII and the elongation stimulatory activity of TFIIF.

By several criteria, the functional properties of SIII more closely resemble those of TFIIF than those of SII. SII has been shown to suppress RNA polymerase pausing in response to intrinsic signals found in a variety of genes, including the human histone H3.3, the Adenovirus major late, and murine adenosine deaminase genes, but it does not appear to produce a general increase in the rate of RNA chain elongation. In contrast, TFIIF does not release polymerase paused at intrinsic pause sites, but it is reported to increase the rate of RNA chain elongation. Under appropriate reaction conditions, RNA polymerase can achieve "physiological, elongation rates of 1200-1500 nt/min in the presence of the TFIIF factor.

Like TFIIF, SIII is capable of stimulating the overall rate of RNA chain elongation under a variety of experimental conditions. In the presence of 500 $\mu$M NTPs and either SIII or recombinant $\beta\gamma$(TFIIF), RNA polymerase elongates RNA chains at about 500 nucleotides/min, consistent with previously reported rates of TFIIF-stimulated elongation measured at 30 °C. SIII is capable of strongly stimulating the rate of elongation of promoter-specific transcripts in the presence of $\beta\gamma$(TFIIF) concentrations that just saturate the initiation reaction.

Present data also indicates that SIII does not promote read-through by polymerase paused at the intrinsic H3.3 pause site. In addition, unlike SII, SIII does not promote cleavage of nascent RNA molecules by paused or stalled RNA polymerase. SII and SIII, therefore, are members of different classes of transcription elongation factors and may perform complementary functions in the regulation of eukaryotic messenger RNA synthesis.

In summary, RNA polymerase transcription factor SIII is defined as a protein, which is composed of subunits with electrophoretic mobilities corresponding to relative molecular masses of ~110 kDa, ~18 kDa and ~15 kDa and which is capable of increasing the rate at which purified RNA polymerase incorporates ribonucleoside triphosphates into RNA chains.

All steps in the purification of SIII are preferably carried out in buffer maintained at about pH 6.5-8.5 unless otherwise indicated.

SIII was purified from a crude nuclear fraction from rat liver, although SIII can be present in any nucleated cell. This crude nuclear fraction contains, in addition to SIII, a large amount of contaminating DNA and RNA as well as many other proteins and enzymes. The procedure summarized below allows separation of SIII from these contaminants. The purified SIII represents <~0.001% (by weight of protein) of the starting material.

Because many nuclear proteins are bound tightly to nucleic acid or other insoluble nuclear components at low ionic strength, in one embodiment of acquiring SIII, nuclei were extracted with a moderate concentration of ammonium sulfate $(NH_4)_2SO_4$ to solubilize as much nuclear protein as possible. Insoluble material was typically removed by centrifugation at >20,000 x g, although other methods known to one skilled in the art can be used. Ammonium sulfate precipitation was used as the preferred initial fractionation step. Following ammonium sulfate fractionation, SIII activity was present in a fraction of protein that was insoluble in about 40% ammonium sulfate. This protein fraction was further fractionated by cation exchange chromatography. Fractions containing SIII activity were pooled, and the SIII further purified using gel exclusion chromatography, or any other procedure which separates proteins according to molecular size. Active fractions from gel exclusion chromatography were pooled and preferably applied to a hydrophobic interaction chromatography resin. The hydrophobic interaction chromatography resin was eluted with a descending concentration gradient of ammonium sulfate. Active fractions were pooled and preferably applied to a cation exchange resin. Active fractions prepared using this or a similar combination of fractionation steps (the order is not necessarily critical) contained three major polypeptides with electrophoretic mobilities corresponding to relative molecular masses of ~110 kDa, ~18 kDa, and ~15 kDa.

These three polypeptides were further purified and separated from one another by reverse phase HPLC. Prior to reverse phase HPLC, the protein was denatured in a solution containing urea, guanidine hydrochloride, trifluoroacetic acid, and acetonitrile. This procedure allowed clean separation of the three polypeptides, which each eluted as a single, sharp peak. When the SIII fraction was loaded onto the reverse phase column without prior denaturation with urea and guanidine hydrochloride, the ~110 kDa polypeptide eluted from the column in multiple broad peaks, which most likely represent different conformers of the polypeptide.

Following reverse phase separation of the three SIII polypeptides, they were mixed together and renatured in various combinations. To renature the SIII polypeptides, volatile solvents from reverse phase fractionation were removed by lyophilization. Dried protein was dissolved in a high concentration of the chaotropic agent guanidine hydrochloride and then rapidly diluted in a pH 7.9 "refolding" buffer containing a moderate concentration of salt, a reducing agent, glycerol, and a low concentration of $ZnSO_4$, which may promote proper folding of one or more of the SIII polypeptides. To remove guanidine hydrochloride, the protein solution was dialyzed for several hours against refolding buffer.

After carrying out the above protocol, various combinations of the renatured ~110 kDa, ~18 kDa and ~15 kDa polypeptides were assayed for their abilities to stimulate transcription elongation by RNA polymerase II. The results of this experiment indicated that all three polypeptides are required for full activity of the SIII protein.

The SIII protein can also be acquired through the utilization of genetic engineering techniques. The methods required to develop an expression system from an isolated protein are known in the art. (Old and Primrose, <u>Principles of Gene Manipulation</u>, 4th. ed. (1989) and Sambrook et al., <u>Molecular Cloning</u>, 2ed., Cold Spring Harbor Laboratory Press, (1989) both of which are incorporated herein by reference).

The cDNAs for the rat derived ~15, ~18 and ~110 kDa subunits have been sequenced (SEQ ID NO: 1, SEQ ID NO: 3, and SEQ ID NO: 5, respectively) as well as the amino acid sequences deduced from those cDNA sequences (SEQ ID NO: 2, SEQ ID NO: 4, and SEQ ID NO: 6, respectively). The methods used are described in Example 2 and are standard procedures in the art. For isolation of cDNAs encoding the ~15 kDa(p15) and the ~18 kDA(p18) subunits, sense and antisense nucleotide sequences based on tryptic peptides of particular subunits were used as primers for PCR. The PCR products were then subcloned to produce plasmid vectors containing partial cDNA sequences. Those cDNAs encoding the complete subunits were obtained by screening rat cDNA libraries using radioactive probes derived from these plasmid vectors by asymmetric PCR. For isolation of cDNAs encoding the ~110 kDa(p110) subunit, a rat cDNA library was screened with degenerate oligonucleotides encoding p110 tryptic peptide sequences to identify candidate cDNAs. Bona fide p110 cDNA clobes were identified by PCR screening, using a primer whose sequence was based on a p110 tryptic peptide sequence. Although these methods worked for their intended purposes, numerous variations which will occur to one skilled in the art, are possible to achieve the same result.

The individual subunits were then produced with M13 expression vectors as described in Example 3. Any of the known expression methods would be suitable substitutions for this vector system, including eukaryotic systems such as baculovirus. The three subunits can then be recombined into the quaternary structure with the expected activity (See Example 4).

Example 5 details the methods used to obtain the whole cDNA for the ~15 kDa human SIII subunit (SEQ ID NO: 7; amino acid sequence, SEQ ID NO: 8), most of the ~ 18 kDa human SIII subunit (SEQ ID NO: 9; amino acid sequence, SEQ ID NO: 10) and partial human ~110 subunit (SEQ ID NO: 11). Briefly probes were prepared from the corresponding rat subunit cDNAs. These probes were then used in PCR or other methods to obtain the human sequences from either a human peripheral blood lymphocyte or human umbilical vein endothelial cell cDNA library. Again, there are various ways known to one skilled in the art to obtain this end. As SIII cDNA has now been found in human peripheral blood lymphocyte, human umbilical vein endothelial cell, rat brain and rat liver cDNA libraries, it is expected that SIII is a ubiquitous protein, involved with transcription in most cells. Therefore, it is expected that SIII cDNA can be isolated from many other mammalian cDNA libraries.

In one embodiment of the invention, probes are made corresponding to sequences of the cDNAs which are complimentary to the mRNA for SIII. These probes can be radioactively or non-radioactively labeled in a number of ways well known to those skilled in the art for use in screening for SIII related proteins or diagnostics. The probes can be made of various lengths. Factors such as stringency and GC content may influence the desired probe length for a particular application. Generally, they can be from 10 nucleotides to the entire nucleic acid coding for a given subunit.

EXAMPLE 1

Isolation, Purification and Detection of SIII (See Fig. 1 and Table I)

Materials

Male Sprague-Dawley rats (200-300 gm) were purchased from SASCO. Unlabelled ultra pure ribonucleoside 5'-triphosphates and dATP were from Pharmacia LKB Biotechnology, Inc. (Piscataway, N.J.). AMP-PNP was obtained from Sigma Chemical Co. (St. Louis, MO) or Pharmacia. [$\alpha$-$^{32}$P] CTP (>650 Ci/mmol) and [$\alpha$-$^{32}$P] ATP (>650 Ci/mmol) were obtained from ICN Biomedicals, Inc. (Costa Mesa, CA). PMSF, antipain, and heparin were from Sigma Chemical Co. Leupeptin was obtained from Boehringer Mannheim Corp. (Indianapolis, IN). Bovine serum albumin (Pentex fraction V) was obtained from ICN ImmunoBiologicals. Glycerol (spectranalyzed grade) was from Fisher Scientific (Pittsburgh, PA). Schwarz/Mann ultrapure sucrose and ammonium sulfate were from ICN Biomedicals, Inc. For reverse-phase chromatography, acetonitrile and trifluoroacetic acid (HPLC/Spectro grade) and urea and guanidine hydrochloride (Sequanal grade) were from Pierce (Rockford, IL).

Chromatography and Buffers

Phosphocellulose (P11) and DEAE-cellulose (DE51) were purchased from Whatman, Inc. (Clifton, N.J.). 4000SW Spherogel TSK, Spherogel TSK phenyl-5-PW, and Spherogel TSK SP5-PW were obtained from Beckman Instruments, Inc. (Fullerton, CA). HPLC was performed using a Beckman System Gold chromatograph. Buffer A was 20 mM HEPES-NaOH, pH 7.9, 1 mM EDTA, 1 mM DTT, 20% (v/v) glycerol, and 0.5 mM PMSF. Buffer B was 50 mM Tris-HCl, pH 7.9, 0.1 mM EDTA, 1 mM DTT, 20% (v/v) glycerol, and 0.5 mM PMSF. Buffer D was 40 mM HEPES-NaOH, pH 7.9, 0.5 mM EDTA, 1 mM DTT, and 10% (v/v) glycerol. Buffer E was 40 mM HEPES-NaOH, pH 7.9, 0.1 mM EDTA, 1 mM DTT, and 10% (v/v) glycerol. Buffer G was 40 mM HEPES-NaOH, pH 7.0, 0.5 mM EDTA, 1 mM DTT, and 10% (v/v) glycerol.

Purification of Transcription Factor SIII

Step 1. Preparation of the Nuclear Extract. A 0.33 M $(NH_4)_2SO_4$ extract of crude rat liver nuclei was prepared from the livers of 300 male Sprague-Dawley rats as previously described (Conaway, J.W. and Conaway, R.C., *J Biol Chem* 264: 2357-2362 (1989)). All further operations were carried out at 4°C.

Step 2: $(NH_4)_2SO_4$ Fractionation. Solid $(NH_4)_2SO_4$ (0.186 g/ml) was added slowly to the nuclear extract. After addition of 1µl 1.0 N NaOH per gram of $(NH_4)_2SO_4$, the suspension was stirred an additional 30 min. The precipitate was collected by centrifugation at 12,000 x g for 45 min and then dissolved in Buffer B containing antipain and leupeptin at 10 µg/ml each (Fraction I).

Step 3: DEAE-cellulose (DE52) Chromatography. Fraction I was diluted with Buffer B to a conductivity equivalent to that of 0.1 M $(NH_4)_2SO_4$ in Buffer B and then centrifuged at 7,500 x g for 15 min. The supernatant was mixed with 1.0 liter of DEAE-cellulose pre-equilibrated with Buffer B containing 0.1 M $(NH_4)_2SO_4$ in a 10.5-cm diameter column. The slurry was allowed to sit for 45 min with occasional stirring and then filtered at 1.0 liter/hour. The column was washed at the same flow rate with Buffer B containing 0.1 M $(NH_4)_2SO_4$ until the eluate contained <0.05 mg/ml protein. Fractions containing protein were pooled and dialyzed against Buffer A to a conductivity equivalent to that of 0.1 M KCl in Buffer A (Fraction II).

Step 4: Phosphocellulose (P11) Chromatography. Fraction II was centrifuged at 4,000 x g for 10 min, and the supernatant was mixed with 800 ml of phosphocellulose pre-equilibrated with Buffer A containing 0.15 M KCl in a 10.5-cm diameter column. The slurry was allowed to sit for 45 min with occasional stirring and then filtered at 500 ml/hour. The column was washed at the same flow rate with Buffer A containing 0.5 M KCl until the eluate contained less than 0.05 mg/ml protein. Transcription activity was eluted stepwise at one packed column volume/hour with Buffer A containing 1.0 M KCl. Fractions of one-fifth column volume were collected, and those containing activity were pooled and dialyzed against Buffer A containing 0.5 M $(NH_4)_2SO_4$ for 2.5 hours (Fraction III).

Step 5: TSK 4000SW HPLC. Solid $(NH_4)_2SO_4$ (0.3 g/ml) was added slowly to Fraction III with stirring. 1 µl of 1.0 N NaOH per gram of $(NH_4)_2SO_4$ was then added, and the suspension was stirred an additional 30 min. The precipitate was collected by centrifugation at 15,000 x g for 90 min and dissolved to a final volume of 5 ml in Buffer G. The solution was dialyzed against Buffer G until the conductivity was equivalent to that of Buffer G in 0.5 M $(NH_4)_2SO_4$ and then centrifuged at 80,000 x g for 20 min. The resulting supernatant was applied to a 4000SW Spherogel TSK HPLC column (21.5 x 600 mm) pre-equilibrated in Buffer G in 0.5 M KCl. The column was eluted at 4 ml/min and 5 ml fractions were collected. Active fractions were pooled (Fraction IV).

Step 6: TSK Phenyl-5-PW HPLC. Fraction IV was diluted with an equal volume of Buffer E containing 2.0 M $(NH_4)_2SO_4$, and then centrifuged at 60,000 x g for 20 min. The resulting supernatant was applied to a Spherogel TSK phenyl-5PW column (21.5 x 150 mm) pre-equilibrated in Buffer E containing 1.0 M $(NH_4)_2SO_4$. Transcription activity was eluted at 5 ml/min with a 500 ml linear gradient from 1.0 M $(NH_4)_2SO_4$ in Buffer E to Buffer E. Ten ml fractions were collected, and the active fractions, which eluted between 0.45 to 0.3 M $(NH_4)_2SO_4$, were pooled and dialyzed against Buffer D containing 0.05 M KCl to a conductivity equivalent to that of Buffer D in 0.1 M KCl (Fraction V).

Step 7: TSK SP-5-PW HPLC. Fraction V was centrifuged at 60,000 x g for 20 min, and the supernatant was applied to a Spherogel TSK SP-5-PW column (7.5 x 75 mm) pre-equilibrated in Buffer D containing 0.1 M KCl. The column was eluted at 1 ml/min with a 50 ml linear gradient from 0.1 to 0.8 M KCl in Buffer D. One ml fractions were collected. Transcription activity eluted between 0.31 and 0.38 M KCl (Fraction VI).

TABLE I

Purification of transcription factor SIII from rat liver

| | FRACTION | PROTEIN (mg) | VOLUME (ml) | ACTIVITY[a] (units) | SPECIFIC ACTIVITY (units/mg) | YIELD (%) |
|---|---|---|---|---|---|---|
| I. | Nuclear Extract | | | | | |
| | 40% $(NH_4)_2SO_4$ fraction | 25,000 | | | | |
| II. | DEAE Cellulose (DE-52) | 13,000 | 3550 | | | |
| III. | Phosphocellulose (P-11) | 240 | 400 | 445,000 | 1,850 | 100[b] |
| IV. | TSK-SW4000 | 70 | 35 | 287,000 | 4,100 | 65 |
| V. | TSK-phenyl-5PW | 4.2 | 60 | 56,300 | 13,400 | 13 |
| VI. | TSK-SP-5PW | 0.35 | 6 | 182,000 | 519,000 | 41 |

[a]Runoff transcription assays performed as described on page 22 using the Eco RI to Nde I fragment from pDN-AdML DNA as template. Following a 30 minute preincubation of RNA polymerase II, transcription factors, and DNA, runoff transcription was carried out for an additional 18 min in the presence of 50 μM ATP, 2 μM UTP, 10 μM CTP, 50 μM GTP, and 10 μCi [α-$^{32}$P] CTP. One unit is the amount of SIII required to reconstitute half maximal runoff transcription.

[b]Overall yield is based on fraction III since SIII activity could not be realiably measured in fractions I and II.

Purification of SIII Polypeptide by Reverse Phase Chromatography

Reverse phase narrow bore HPLC was performed using an Ultra-fast Micro Protein Analyzer (Michrom BioResources, Pleasanton, CA). An aliquot (~50 μg) of SIII (Fraction VI) was diluted 1:1 with "magic mix", (Nugent, et al., *J Chromatography* 443: 381-397 (1988)), denaturant (4.0 M guanidine hydrochloride, 4.0 M urea, 7.5% acetonitrile, 0.15% TFA), and 0.2% Zwittergent ZC-8 (Calbiochem) and applied to a 2.1 x 50 mm PLRP-S (1OOOÅ pore size; 8µ particle size) reverse phase HPLC column (Michrom BioResources) pre-equilibrated at 54°C in 20% eluent B (90% acetonitrile, 0.09% TFA, and 0.03% Zwittergent ZC-8) and 80% eluent A (2% acetonitrile, 0.1% TFA, and 0.03% Zwittergent ZC-8). The column was developed at 0.4 ml/min with a 22 min linear gradient from 20% to 75% eluent B. Peaks of absorbance at 220 nm were collected manually.

Recovery of SIII Activity Following Reverse Phase HPLC

Fractions from reverse phase narrow bore HPLC were lyophilized until just dry using a Savant Speed-Vac (Savant Instruments, Farmingdale, N.Y.). Dried protein was resuspended in 5 µl of 6.0 M guanidine hydrochloride and left on ice for 30 min. Aliquots were then diluted to 50 µl with renaturation buffer (40 mM HEPES-NaOH, pH 7.9, 0.1 M KCl, 2 mM DTT, 50 µM $ZnSO_4$, 0.1 mM EDTA, and 10% (v/v) glycerol) and left on ice an additional 90 min. Aliquots were then dialyzed for 2 hours against renaturation buffer lacking EDTA and DTT and stored at -80°C.

Preparation of RNA Polymerase II and Transcription Factors

RNA polymerase II (Serizawa, et al., *Proc Natl Acad Sci USA* 89: 7476-7480 (1992)) the native rat TATA factor τ (Conaway et al., *J Biol Chem* 265: 7552-7558 (1990)) and transcription factor δ (BTF2) (Conaway, R.C. and Conaway, J.W., *Proc Natl Acad Sci USA* 86: 7356-7360 (1989); and Conaway, et al., *J Biol Chem* 267: 10142-10148 (1992)) were purified from rat liver as previously described. Recombinant yeast TFIID was expressed and purified as described (Conaway, et al., *J Biol Chem* 266: 7804-7811 (1991)) from bacterial strain N5151 containing the plasmid pASY2D (Schmidt, et al., *Proc Natl Acad Sci USA* 86: 7785-7789 (1989)). Recombinant rat α(TFIIB) (Tsuboi, et al., *Nucleic Acids Res* 20: 3250 (1992)) and recombinant human TFIIE (Peterson, et al., *Nature* 354: 369-373 (1991)) were prepared as described, except that the 56 kDa subunit of TFIIE was expressed in BL21(DE3). Recombinant βγ(TFIIF) was purified by phosphocellulose chromatography (Conaway, J.W. and Conaway, R.C., *J Biol Chem* 264: 2357-2362 (1989)) of whole cell extracts prepared from SF21 cells co-infected with recombinant baculoviruses encoding the βγ(TFIIF) subunits, human RAP74 (Aso, et al., *Nature* 355: 461-464 (1992); and Finkelstein, et al., *Nature* 355: 464-467 (1992)) and rat RAP30 (Garrett, et al., *J Biol Chem* 267: 23942-23949 (1992)). Recombinant viruses were constructed using the BacPAK6 baculovirus expression system (Clontech Laboratories, Inc., Palo Alto, CA).

Assay of Runoff Transcription

Unless indicated otherwise, preinitiation complexes were assembled as described (Conaway, et al., *J Biol Chem* 262: 8293-8297 (1987)) by preincubation of 100 ng of Nde I-digested pDN-AdML, (Conaway R.C. and Conaway, J.W., *J Biol Chem* 263: 2962-2968 (1988)) or 100 ng of Nde I-digested $pN_4$ (Lorch, et al., *Cell* 49: 203-210 (1987)) and approximately 10 ng of recombinant α(TFIIB), 10 ng of recombinant βγ-(TFIIF), 7 ng of recombinant human TFIIE, 40 ng of δ(BTF2) (Fraction VI), 60 ng τ (Fraction V) or 50 ng of recombinant yeast TFIID (AcA 44 fraction), and 0.01 unit of RNA polymerase II. Transcription was initiated by addition of 7 mM $MgCl_2$ and 50 µM ATP, 2 µM UTP, 10 µM CTP, 50 µM GTP and 10 µCi ($\alpha$-$^{32}$P] CTP. After incubation at 28°C for 18 min runoff transcripts were analyzed by electrophoresis through 6% polyacrylamide/7.0 M urea gels. Transcription was quantitated by densitometry of autoradiograms using an LKB UltroScan XL laser densitometer.

Sucrose Gradient Sedimentation

Sedimentation was performed in 2 ml linear sucrose gradients (15-30% [v/v] containing 20 mM HEPES-NaOH, pH 7.9, 1 mM EDTA, 1 mM DTT, and 0.4 M KCl). Centrifugation was carried out at 55,000 rpm and 4°C in the TLS55 rotor of a Beckman TL1OO ultracentrifuge. Fractions (2 drops) were collected from the bottom of tubes through a 20-gauge needle.

Protein Determination

Protein concentrations were determined using the protein dye assay (Bio-Rad Laboratories, Hercules, CA) with bovine serum albumin as the standard.

Detection of Transcription Factor SIII

SIII-dependent transcriptional stimulation is readily detected if any of the four ribonucleoside triphosphates are present at limiting concentrations. This property of SIII was exploited to purify it to apparent homogeneity from rat liver nuclear extracts. SIII was assayed by its ability to reconstitute synthesis of a 250-nucleotide runoff transcript from the core region of the AdML Promoter in the presence of a limiting concentration of UTP and saturating amounts of RNA polymerase II, initiation factors $\alpha$(TFIIB), $\beta\gamma$-(TFIIF),$\delta$(BTF2/TFIIH), and $\epsilon$(TFIIE), and either the native rat TATA factor $\tau$ or recombinant yeast TFIID. The standard template was pDN-AdML (Conaway, R.C. and Conaway, J.W., *J Biol Chem* 263: 2962-2968 (1988)), which includes AdML core promoter sequences, but which lacks the upstream sequences that mediate stimulation of transcription by USF/MLTF.

SIII was purified to near homogeneity from a 0.33 M $(NH_4)_2SO_4$ extract of crude rat liver nuclei by ammonium sulfate fractionation, followed by chromatography on successive DEAE-cellulose, phosphocellulose, TSK 4000SW, TSK phenyl-5-PW, and TSK SP-5-PW columns. SIII activity was first reliably measured in the phosphocellulose fraction (Fraction III). Approximately 350 $\mu$g of SIII (Fraction VI) can be purified from ~3 kg of rat liver. The overall yield from Fraction III was approximately 40%. The more than 100% yield of SIII activity on TSK SP-5-PW was most likely due to removal of inhibitors present in Fraction V.

SIII is a multisubunit protein composed of ~110, ~18, and ~15 kDa polypeptides. Analysis of the TSK SP-5-PW column fractions by SDS-polyacrylamide gel electrophoresis revealed that polypeptides with apparent molecular masses of approximately 110, 18, and 15 kDa co-chromatographed with transcriptional stimulatory activity and accounted for more than 90% of the protein in the active fractions. Transcriptional stimulatory activity did not elute from TSK SP-5-PW in a symmetrical peak. The bulk of activity was recovered in fractions 25 and 26, but significant activity eluted as a shoulder on the leading edge of this peak. The two small polypeptides appeared to be present in higher molar amounts (relative to the large polypeptide) in fractions 25 and 26 than in the earlier eluting fractions. In addition, it was observed that the ~110, ~18, and ~15 kDa polypeptides also co-chromatographed with transcriptional activity when SIII was analyzed by hydrophobic interaction chromatography on TSK phenyl-5-PW (data not shown).

To determine which polypeptides were required to reconstitute SIII activity, the protein in an aliquot of fraction 26 from the TSK SP-5-PW column was denatured and fractionated by reverse phase HPLC. The ~110, ~18, and ~15 kDa polypeptides were each recovered in apparently homogeneous form. The polypeptides were renatured alone or in combination with other polypeptides and assayed for activity. Transcriptional activity was recovered only when all three polypeptides were combined and renatured together. It could not be reconstituted with any single polypeptide renatured independently or with any combination of independently renatured polypeptides. In addition, transcriptional activity could not be recovered when pairs of polypeptides were renatured together. However, in studies using recombinant subunits (See Example 4) a slight stimulation of transcription was observed with p110 alone; the combination of p110 and p15 had somewhat more activity and maximal activity depended on the presence of the p110, p15 and p18 subunits. Thus, it appears that p110 alone exhibits a low, basal level of transcription while both the p18 and p15 subunits strongly stimulate this activity.

As measured by TSK 4000SW size exclusion HPLC, SIII has a Stokes radius of ~47Å. By sucrose gradient sedimentation, SIII has a sedimentation coefficient of ~6 S. Assuming a partial specific volume of 0.725 ml/g, SIII has an apparent native molecular mass determined by the method of Siegel and Monty (Siegel, L.M. and Monty, K.J., *Biochim Biophys Acta* 112: 346-362 (1966)) of ~140 kDa, consistent with SIII being a heterotrimer composed of a single copy each of the ~110, ~18, and ~15 kDa polypeptides.

EXAMPLE 2

Isolation of cDNA Clones Encoding Rat SIII Protein

Method for Generating cDNA From ~15 kDa SIII Subunit

Approximately 300 pmol of the SIII p15 subunit, isolated by reverse phase HPLC, was digested with trypsin. The $NH_2$ terminal sequences of four tryptic peptides (I-IV), determined by automated Edman microsequencing, were as follows: I, $NH_2$-LISSDGHEFIVKR-COOH (SEQ ID NO: 12);II, $NH_2$-AMLSGPGQ-FAENETNEVNFR-COOH (SEQ ID NO: 13); III, $NH_2$-VCMYFTYK-COOH (SEQ ID NO: 14);IV, $NH_2$-YTNS-STEIPEFPIAPEIALELLMAANFLD-COOH (SEQ ID NO:15). A partial cDNA encoding residues 51-97 of the SIII p15 polypeptide was isolated by polymerase chain reaction (PCR) using as primers the sense and antisense degenerate oligonucleotides 5'-CARTTYGCNGARAAYGARAC-3' (SEQ ID NO:16) and 5'-GGNGCDATNGGRAAYTCNGG-3' (SEQ ID NO:17) encoding residues 8 through 14 of tryptic peptide II and residues 9 through 15 of tryptic peptide IV, respectively. PCR was performed for 30 cycles of 1 min at 94°C, 1 min at 46°C, and 2 min at 72°C with 1.5 mM $MgCl_2$, 0.25 mM dNTPs, 2.5 units of Taq polymerase, 0.02 $A_{260}$ unit of each primer, and ~6 x $10^6$ pfu of a rat liver λgt11 cDNA library (Clontech). PCR products encoding SIII p15 polypeptide sequences were identified by Southern blotting, using as probe the 5'-$^{32}$P-labeled degenerate oligonucleotide 5'-ACNAAYGARGTNAAYTTYMG-3' (SEQ ID NO:18), which encodes residues 14 through 20 of tryptic peptide II, isolated by preparative polyacrylamide gel electrophoresis, and subcloned by blunt end ligation into pBluescript KS(-). Bacteria harboring a recombinant plasmid (pKGl) carrying the partial SIII p15 cDNA were identified by colony hybridization using the same 5'-$^{32}$P-labeled degenerate oligonucleotide as probe. cDNAs encoding the complete SIII p15 polypeptide were obtained by screening rat liver and rat brain λ ZAP II cDNA libraries (Stratagene, La Jolla, CA) with an internally labeled, single stranded DNA probe synthesized by asymmetric PCR, using pKG1 as template. pBluescript SK(-) phagemids containing cDNA inserts were rescued with helper phage and sequenced by the dideoxy chain termination method using a Sequenase kit (United States Biochemical Corp., Cleveland, OH). Two overlapping clones were combined to generate the full length rat SIII p15 coding sequence (SEQ ID NO:1, nucleotide sequence; SEQ ID NO:2, amino acid sequence).

Method for Generating cDNA From ~18 kDa SIII Subunit

Approximately 300 pmol of the SIII p18 subunit was isolated by reverse phase HPLC (Bradsher, et al., *J Biol Chem* 268: 25587-25593 (1993a)). After reduction, S-carboxyamidomethylation, and digestion with trypsin, the resultant mixture was further fractionated by microbore HPLC. Optimal peptides were determined by differential UV absorbance and matrix-assisted laser desorption mass spectrometry (Lasermat; Finnigan-MAT, San Jose, CA) and submitted to automated Edman microsequencing (Lane, et al., *J Prot Chem* 10: 151-160 (1991)). The N-terminal sequences of two tryptic peptides (I and II) were obtained and were as follows: I, LYKDDQLLDDGKTLGECGFTSQTARPQ(A) (P) (SEQ ID NO: 19) and II, ADDTFEALRIEPFSSPPELPDVMKPQDSG[G] (S)AN[E] (SEQ ID NO:20). A partial p18 cDNA was isolated from a rat liver λgt11 cDNA library (Clontech) by PCR, using as primers the sense and antisense degenerate oligonucleotides 5'-TNTA(Y)AA(R)GA(Y)GA(Y)CA(R)(Y)T-3' (SEQ ID NO:21) and 5'-TGNGG(Y)-TTCATNAC(R)TCNGG-3' (SEQ ID NO:22), which encode portions of tryptic peptide I and tryptic peptide II, respectively (R is A or G; Y is C or T; N is A, C, G, or T). PCR was performed for 30 cycles of 1 min at 94°C, 1 min at 46°C, and 2 min at 72°C with 1.5 mM $MgCl_2$, 0.25 mM dNTPs, 2.5 units of *Taq* polymerase, and 0.02 $A_{260}$ unit of each primer. PCR products encoding SIII p18 polypeptide sequences were identified by Southern blotting using as probe the 5'-$^{32}$P-labeled degenerate oligonucleotide 5'-GCNGA(Y)GA(Y)ACNTT(Y)GA(R)GC-3' (SEQ ID NO:23) which encodes residues 1-7 of tryptic peptide I, isolated by preparative polyacrylamide gel electrophoresis and subcloned by blunt-end ligation into pBluescript KS(-). Bacteria harboring the recombinant plasmid (pKG2) carrying the partial SIII p18 cDNA were identified by colony hybridization using the same 5'-$^{32}$P-labeled degenerate oligonucleotide as probe. A cDNA encoding the complete SIII p18 polypeptide was obtained by screening a rat brain λZAP II cDNA library (Stratagene, La Jolla, CA) with an internally labeled, single-stranded DNA probe synthesized by asymmetric PCR (McCabe, P.C., *PCR Protocols: A Guide to Methods and Applications.* Innis, et al., eds. (San Diego: Academic Press), pp. 76-83 (1990)) using pKG2 as template. Bluescript SK(-) phagemids containing cDNA inserts were rescued with VCS-M13 interference-resistant helper phage (Stratagene) and sequenced by the dideoxy chain-termination method using a Sequenase kit (United States Biochemical Corp.). The SIII p18 nucleotide sequence is given in SEQ ID NO: 3, and the corresponding amino acid sequence is listed in SEQ ID NO:4.

Method for Generating cDNA From ~110 kDA SIII Subunit

Approximately 300 pmol of the SIII p110 subunit was isolated by reverse phase HPLC (Bradsher, et al., *J Biol Chem* 268: 25587-25593 (1993)). After reduction, S-carboxyamidomethylation, and digestion with trypsin, the resultant mixture was further fractionated by microbore HPLC. Optimal peptides were determined by differential UV absorbance and matrix-assisted laser desorption mass spectrometry (Lasermat; Finnigan-MAT, San Jose, CA), and then submitted to automated Edman microsequencing, (Lane, et al., *J Prot Chem* 10: 151-160 (1991)). The $NH_2$ terminal sequence of two trypyic peptides (I and II) were as follows: I, DVPQQEEEAEGNYQESWQASGSQPY(Y) (P)EHR (SEQ ID NO:24); II, ANENKSDKLQPAGAEPTRP (SEQ ID NO: 25). Two oligonucleotide "guessmer" probes were designed according to human codon usage bias. At some positions, inosine (I), was used instead of a mixture of four nucleotides. Primer 1, which is the complement of a sequence encoding amino acids 1-15 of tryptic peptide I, was 5'TCCTGGTAGTTICCTCIG CCTCCTCCTCCTGCTGIGGIACGTC (SEQ ID NO:26), and Primer 2, which is the complement of a sequence encoding amino acids 10-17 of tryptic peptide two, was 5'CGGATCGTIGG-(T/C) TCIGCICCIGCIGG(T/C)TG (SEQ ID NO:27).

A λGEM2 library constructed from size selected (>2kb) cDNA derived from rat brain (from Dr. Roger Wiegand, Monsanto Corp., Saint Louis, Mo.) was screened with 5'-$^{32}$P-labeled Primer 1. Hybridization was at 37°C for 20 hours in 6X standard saline citrate (SSC)/1X Denhardt's solution/0.05% sodium pyrophosphate containing denatured salmon testes DNA at 100 µg/ml. Sixty-nine clones were isolated from 5 x $10^5$ plaques. Phage clones derived from oligonucleotide screening were further screened by the polymerase chain reaction (PCR) method, using a T7 primer, CGTAATACGACTCACTATAGGG, (SEQ ID NO:28), which hybridizes to a sequence in the λGEM2 vector, and Primer 2, respectively, as sense and anti-sense primers. PCR reactions were performed for 30 cycles of 1 min at 94°C, 1 min at 55°C, and 2 min at 72°C. PCR reactions performed with eight of 69 phage clones yielded discrete products; the clone yielding the largest PCR product was selected and sequenced by the dideoxy chain termination method using a Sequenase kit. The SIII p110 nucleotide sequence is given in SEQ ID NO:5 and the corresponding amino acid sequence is listed in SEQ ID NO:6.

EXAMPLE 3

Expression of the Rat SIII protein in *E. Coli*

Expression of the SIII p15 Polypeptide in *E. coli.*

Overexpression of the SIII p15 polypeptide in *E. coli* was accomplished using an M13mpET bacteriophage expression system. The entire SIII p15 coding sequence was introduced into M13mpET, which contains the complete pET T7 transcription/expression region. A 100-ml culture of *E. coli* strain JM109(DE3) (Promega) was grown to an $OD_{600}$ of 0.6 in SOB medium containing 2.5 mM $MgCl_2$ at 37°C with gentle shaking. Cells were infected with M13mpET carrying the full-length SIII p15 cDNA at a multiplicity of infection of 10-20. After an additional 2 hours at 37°C, cells were induced with 0.4 mM isopropyl β-D-thiogalactoside, and the culture was incubated an additional 2.5 hours. Cells were harvested by centrifugation at 2000 x g for 10 min at 4°C, and inclusion bodies were prepared as previously described (Lin, K. & Cheng, S. *BioTechniques* 11: 748-753 (1991)), except that DNase and RNAse treatments were omitted. Inclusion bodies were solubilized by resuspension in 2 ml of ice-cold 50 mM Tris-HCl (pH 8.0) containing 6 M guanidine hydrochloride. The resulting suspension was clarified by centrifugation at 50,000 x g for 20 min at 4°C.

Expression of Histidine-tagged SIII p18 Polypeptide in *E. coli.*

Overexpression of histidine-tagged p18 polypeptide was accomplished using an M13mpET bacteriophage expression system (Tan, et al., *BioTechniques* 16:824-828 (1994)). To make the expression vector m13mpET-6Hp18, which encodes p18 with an 10 amino acid extension with the sequence MHHHHHHNVD (SEQ ID NO:29), the entire open reading frame encoded by the SIII p18 cDNA was subcloned into the M13mpET-6H bacteriophage vector, (Tan, et al., *BioTechniques* 16: 824-828 (1994)). A 100 ml culture of *E. coli* strain JM109(DE3) was grown to an $OD_{600}$ of 0.6 in SOB medium containing 2.5 mM $MgCl_2$ at 37°C with gentle shaking. Bacteria were infected at a multiplicity of infection of 10-20. After an additional 2 hours at 37°C, cells were induced with 0.4 mM isopropyl β-D-thiogalactoside, and the culture was incubated an additional 2.5 hours. Bacteria were harvested by centrifugation at 2000 x g for 10 min at 4°C, and inclusion bodies were prepared as described, (Lin, K. and Cheng, S. *BioTechniques* 11: 748-753 (1991)), except that RNAase and DNAase treatments were omitted. Inclusion bodies were solubilized by resuspension in 2 ml ice-cold buffer containing 6 M guanidine hydrochloride, 40 mM Tris-HCl (pH 7.9), 10 mM imidazole (pH 7.9), 0.5 mM PMSF, and 1 mM DTT. The resulting suspension was clarified by centrifugation at 50,000 x g for 20 min at 4°C and applied to a column containing 1 ml ProBond™ Metal-Binding Resin (Invitrogen) equilibrated with the same buffer. The column was washed with 10 ml of buffer containing 5.8 M guanidine hydrochloride, 40 mM Tris-HCl (pH7.9), 40 mM imidazole (pH 7.9), 0.5 mM PMSF, and 1 mM DTT. Histidine-tagged p18 was then eluted with 3 ml of buffer containing 4 M guanidine hydrochloride, 40 mM Tris (pH 7.9), 300 mM imidazole (pH 7.9), 0.5 mM PMSF, and 1 mM DTT.

Expression of Histidine-tagged SIII p110 Polypeptide in *E. coli.*

Overexpression of histidine-tagged p110 was accomplished using an M13mpET bacteriophage expression system (Tan, et al., *BioTechniques* 16: 824-828 (1994)). To make the expression vector ml3mpET-6Hp110, which encodes p110 with an 10 amino acid extension with the sequence MHHHHHHNVD (SEQ ID NO:30), the entire open reading frame encoded by the p110 cDNA was subcloned into the M13mpET-6H bacteriophage vector. A 100 ml culture of *E. coli* strain JM 109(DE3) was grown to an $OD_{600}$ of 0.6 in SOB medium containing 2.5 mM $MgCl_2$ at 37°C with gentle shaking. Bacteria were infected at a multiplicity of infection of 10-20. After an additional 2 hours at 37°C, cells were induced with 0.4 mM isopropyl $\beta$-D-thiogalactoside, and the culture was incubated an additional 2.5 hours. Bacteria were harvested by centrifugation at 2000 x g for 10 min at 4°C, and inclusion bodies were prepared as described (Lin, K. and Cheng, S. *BioTechniques* 11: 748-753 (1991)) except that the RNAase and DNAase treatments were omitted. Inclusion bodies were solubilized by resuspension in 2 ml ice-cold buffer containing 6 M guanidine hydrochloride, 40 mM Tris-HCl (pH 7.9), 10 mM imidazole (pH 7.9), 0.5 mM PMSF, and 1 mM DTT. The resulting suspension was clarified by centrifugation at 50,000 x g for 20 min at 4°C and applied to a column containing 1 ml ProBond™ Metal-Binding Resin (Invitrogen) equilibrated with the same buffer. The column was washed with 10 ml of buffer containing 5.8 M guanidine hydrochloride, 40 mM Tris-HCl (pH 7.9), 40 mM imidazole (pH 7.9), 0.5 mM PMSF, and 1 mM DTT. Histidine-tagged pl8 was then eluted with 3 ml of buffer containing 4 M guanidine hydrochloride, 40 mM Tris (pH 7.9), 300 mM imidazole (pH 7.9), 0.5 mM PMSF, and 1 mM DTT.

EXAMPLE 4

Transcription Activity of Recombinant SIII

To reconstitute SIII, ~100 ng recombinant histidine-tagged p18 and ~ 500 ng recombinant histidine-tagged p110, both in a solution of 4 M guanidine hydrochloride, 40 mM Tris-HCl (pH7.9), 300 mM imidazole (pH 7.9), 0.5 mM PMSF, and 1 mM DTT, were added to ~ 100 ng lyophilized recombinant p15, which had been prepared as described, (Garrett, et al., *Proc Natl Acad Sci USA* 91: 5237-5241 (1994)). This mixture was diluted ~ 15 fold with a buffer containing 40 mM HEPES-NaOH (pH 7.9), 100 mM KCl 2 mM DTT, 50 $\mu$M $ZnSO_4$, 0.1 mM EDTA, and 10% (v/v) glycerol and incubated 90 min on ice. Renatured proteins were dialyzed for 2 hours against the same buffer lacking EDTA and DTT. In some experiments, p18, p15, or p110 were omitted from reconstitution mixtures (See Fig. 3). Renatured proteins were assayed for SIII transcriptional activity as described (Bradsher, et al., *J Biol Chem* 268: 25594-25603 (1993b)); Garrett, et al., *Proc Natl Acad Sci USA* 91: 5237-5241 (1994)).

Maximal stimulation of the rate of accumulation of full length runoff transcripts is dependent on the presence of p110, p15, and p18. A small stimulation of transcription is observed with p110 alone, but neither p15, p18, nor a combination of p15 and p18 detectably stimulate transcription. Thus, p15 and p18 function as positive regulators of p110 activity.

EXAMPLE 5

Isolation of cDNA Clones Encoding Human SIII Protein

Isolation of a cDNA clone encoding human SIII p15

A λZAPII library constructed with cDNA derived from peripheral blood lymphocytes (obtained from Drs. J. Culpeper and F. Lee, DNAX Research Institute, Inc., Palo Alto, CA) was screened with an internally labelled, single stranded probe containing rat p15 cDNA sequences. The probe was prepared by asymmetric PCR using as template a DNA fragment containing the entire rat p15 cDNA. Asymmetric PCR reactions were performed for 30 cycles of 1 min at 94°C, 1 min at 47°C, and 1 min at 72°C with 1.5 mM MgCl2, 50 $\mu$M dATP, 50 $\mu$M dGTP, 50 $\mu$M dTTp, 100 $\mu$Ci [$\alpha$-$^{32}$P]dCTP (3000 Ci/mmole) and an anti-sense oligonucleotide primer, GCA GCG GAT CCT CAA CAA TCT AGG AAG TTC G (SEQ ID NO:31), which contains sequences derived from the 3' end of the rat p15 cDNA. Hybridization was at 50°C for 20 hrs in 5X standard saline citrate (SSC)/5X Denhardt's solution/0.1% SDS/10% dextran sulfate/100 mM $NaHPO_4$/1 ml/ml bovine serum albumin/100 $\mu$g/ml herring sperm DNA. Bluescript SK(-) phagemids containing cDNA inserts were rescued with VCS-M13 interference-resistant helper phage (Stratagene) and sequenced by the dideoxy chain-termination method using a Sequenase kit (United States Biochemical Corp.).

The human SIII p15 nucleotide sequence is given in SEQ ID NO:7 and the predicted amino acid sequence is listed in SEQ ID NO: 8. The human p15 cDNA encodes a protein with a predicted amino acid sequence identical to that of rat p15.

These findings indicate that the human p15 mRNA is expressed in peripheral blood lymphocytes.

Isolation of a partial cDNA clone encoding human SIII p18

Oligonucleotide I: GCA ACG TCG ACA TGG ACG TGT TTC TCA TGA T (SEQ ID NO:32), and II: GCA GCG GAT CCT CAC TGC ACA GCT TGT TCA T (SEQ ID NO:33) containing DNA sequences encoding the rat SIII p18 amino terminus and carboxyl terminus, respectively, were used in PCR reactions to isolate a partial human SIII p18 cDNA from a λZAPII human peripheral blood lymphocyte cDNA library, which was obtained from J. Culpepper and F. Lee (DNAX Research Institute, Palo Alto, CA). PCR reactions were performed for 30 cycles of 1 min at 94°C, 1 min at 46°C, and 2 min at 72°C with 1.5 mM $MgCl_2$, O.25 mM dATP, 0.25 mM dCTP, 0.25 mM dGTP, 0.25 mM dTTP, 2.5 units of Taq polymerase, 0.02 A260 units of each primer and ~4 x $10^6$ pfu of the library.

PCR products containing p18 inserts were identified by Southern blot hybridization using as probe 5'-α-$^{32}$P labelled oligonucleotide III, which encodes residues 81-87 of the rat SIII p18 subunit. Appropriately sized PCR products were purified by polyacrylamide gel electrophoresis, and subcloned by blunt-end ligation into pBluescript KS(-). Bacteria harboring a recombinant plasmid carrying the partial human SIII p18 cDNA were identified by colony hybridization using oligonucleotide III: GCN GA(C/T) GA(C/T) ACN TT(C/T) GA(G/A) GC (SEQ ID NO:34) as probe.

The resulting cloned PCR product was sequenced by the dideoxy chain termination method using a Sequenase kit. The partial human p18 cDNA encodes a protein with a predicted amino acid that is almost identical to that of rat p18. The partial human SIII p18 nucleotide sequence is given in SEQ ID NO:9 and the corresponding amino acid sequence is listed in SEQ ID NO:10.

These findings indicate that the human p18 mRNA is expressed in peripheral blood lymphocytes.

Isolation of a human cDNA encoding the p110 subunit of transcription factor SIII.

cDNAs encoding the p110 subunit of human SIII were obtained by screening 1 x $10^5$ colonies of a pSPORT-1 human umbilical vein endothelial cell (HUVEC) cDNA library with a $^{32}$P-labelled probe comprised of the entire rat p110 coding sequence. The library was a gift of Kenji Fukudome (Oklahoma Medical Research Foundation Oklahoma City, OK). Prehybridization was carried out for 2 hours at 42°C in 4X SSC, 0.1% SDS, 40% formamide, 20 mM Tris-HCl (pH 7.5), 4% dextran sulfate, 1X Denhardt's solution, and 100 µg/ml salmon sperm DNA. Hybridization was carried out for 16 hours at 42°C in the above buffer containing radiolabelled probe. Colony lifts were washed twice for 15 min at room temperature in 2X SSC containing 0.1% SDS. Positive bacterial colonies were purified by two additional rounds of screening under the same conditions except that hybridization was carried out at 45°C, and the two washes were performed at 45°C for 20 min each. Positive clones containing the largest inserts were sequenced. Preliminary DNA sequence analysis indicates that the rat and human p110 cDNAs are highly similar, but less highly conserved than the rat and human p15 and p18 subunits. The partial human SIII p110 nucleotide sequence is given in SEQ ID NO:11.

EXAMPLE 6

SIII Effect on Transcription Rate

Materials

Unlabelled ultrapure ribonucleoside 5'-triphosphates and dATP were from Pharmacia LKB Biotechnology, Inc. AMP-PNP was obtained from Sigma or Pharmacia. [α-$^{32}$P] CTP (>650 Ci/mmol) and [α-$^{32}$P] UTP (>650 Ci/mmol) were obtained from ICN. Sarkosyl and heparin were from Sigma. Bovine serum albumin (Pentex fraction V) was obtained from ICN Immunobiologicals. Agarose (GenAR) was from Mallinckrodt (St. Louis, MO).

Preparation of RNA polymerase II and transcription factors

Transcription factor SIII was purified from rat liver nuclear extracts as described in Example 1. Recombinant yeast TFIID was expressed and purified as described (Conaway, et al., *J Biol Chem* 266: 7804-7811 (1991)) from bacterial strain N5151 containing the plasmid pASY2FD (Schmidt, et al., *Proc Natl Acad Sci USA* 86: 7785-7789 (1989)). Recombinant rat TFIID was expressed in *Escherichia coli* using T7-expression vector pET-lla (Novagen) and was purified from extracts by chromatography on DEAE Sepharose and heparin Sepharose as described (Hoey, et al., *Cell* 61: 1179-1186 (1990)). Recombinant rat α(TFIIB) (Tsuboi, et al., *Nucleic Acids Res* 20: 3250 (1992)) and recombinant human TFIIE (Peterson, et al., *Nature* 354: 369-373 (1991)) were prepared as described, except that the 56 kDa subunit of TFIIE was expressed in BL21(DE3). Recombinant βγ(TFIIF) was purified by phosphocellulose chromatography (Conaway, J.W. and Conaway, R.C., *J Biol Chem* 264: 2357-2362 (1989)), of whole cell extracts prepared from Sf21 cells co-infected with recombinant baculoviruses encoding human RAP74 (Aso, et al., *Nature* 355: 461-464 (1992); Finkelstein, et al., *Nature* 355: 464-467 (1992)) and rat RAP30 (Garrett, et al., *J Biol Chem* 267: 23942-23949 (1992)). Recombinant viruses were constructed using the BacPAK6 baculovirus expression system (Clontech). RNA polymerase II (Serizawa, et al., *Proc Natl Acad Sci USA* 89: 7476-7480 (1992)), the native rat TATA factor τ (Conaway, et al., *J Biol Chem* 265: 7552-7558 (1990)), and transcription factor δ(BTF2) (Conaway, R.C. and Conaway, J.W., *Proc Natl Acad Sci USA* 86: 7356-7360 (1989)); Conaway, et al., *J Biol Chem* 267: 10142-10148) (1992)) were purified from rat liver as previously described.

Assay of runoff transcription

Unless indicated otherwise, preinitiation complexes were assembled as described (Conaway, et al., *J Biol Chem* 262: 8293-8297 (1987)) by preincubation of 100 ng of Nde I-digested pDN-AdML (Conaway, R.C. and Conaway, J.W., *J Biol Chem* 263: 2962-2968 (1988)), 100 ng of Nde I-digested $pN_4$ (Lorch, et al., *Cell* 49: 203-210 (1987)), or 10 ng of the Eco RI to Nde I fragment from pDN-AdML, and approximately 10 ng of recombinant α(TFIIB), 10 ng of recombinant βγ(TFIIF), 7 ng of recombinant human TFIIE, 40 ng of δ-(BTF2) (Fraction VI), 60 ng τ (Fraction V) or 50 ng of recombinant yeast TFIID (AcA 44 fraction), and 0.01 unit of RNA polymerase II. Transcription was initiated by addition of 7 mM $MgCl_2$ and ribonucleoside triphosphates in the concentrations noted below. After incubation at 28 °C, runoff transcripts were analyzed by electrophoresis through 6% polyacrylamide/7.0 M urea gels. Transcription was quantitated by densitometry of autoradiograms using an LKB UltroScan XL laser densitometer.

Isolation of DNA fragments

DNA fragments were excised from 1.5% agarose gels and purified using GENECLEAN II (BIO 101 Inc.) according to the manufacturer's instructions.

Determination of Transcription Rate

AMP-PNP is used less well than ATP as a substrate for RNA chain elongation (Ernst, et al., *Mol Cell Biol* 3: 2172-2179 (1983)). At AMP-PNP concentrations <10 µM, full length runoff transcripts initiated from the AdML promoter do not accumulate, but there is a substantial accumulation of shorter products. At AMP-PNP concentrations >10 µM, significant synthesis of full length runoff transcripts is observed. Furthermore, the short RNA products formed in the presence of low concentrations of AMP-PNP can be chased into full length runoff transcripts if ATP is added to reaction mixtures after the addition of heparin, which inhibits promoter-specific transcription initiation but not elongation of previously initiated RNA chains (Conaway, R.C. and Conaway, J.W., *J Biol Chem* 265: 7559-7563 (1990); Egly, et al., *EMBO J* 3: 2363-2371 (1984)). Regardless of the AMP-PNP concentration present in reaction mixtures prior to the ATP chase, roughly equivalent levels of runoff transcripts were synthesized from the AdML promoter following addition of heparin and ATP. Thus, the short transcripts formed in the presence of low AMP-PNP concentrations and in the absence of SIII are not terminated, but, rather, appear to be contained in transcriptionally active but stalled RNA polymerase II elongation complexes.

To determine whether the effect of SIII is a general or whether it is specific for reactions in which AMP-PNP replaces ATP as a substrate for RNA chain elongation, runoff transcription assays were performed, with and without SIII, in which the concentration of each of the four ribonucleoside triphosphates was lowered individually in the presence of fixed concentrations of the other three ribonucleoside triphosphates.

SIII is able to stimulate synthesis of accurately initiated transcripts when reactions are carried out with limiting concentrations of any of the ribonucleoside triphosphates. During a 30 min incubation, maximal stimulation of transcription was observed when the limiting ribonucleoside triphosphate was included in reaction mixtures at concentrations less than 2 µM. The extent to which SIII stimulates accumulation of full length products appears to differ depending on which ribonucleoside triphosphate is limiting.

Examination of the kinetics of accumulation of full-length runoff transcripts revealed that SIII significantly increases the rate of incorporation of ribonucleoside triphosphates into growing RNA chains. (See Fig. 2) An investigation of the effect of SIII on runoff transcription was also carried out in the presence of limiting UTP. Under these conditions, full-length runoff transcripts synthesized in the absence of SIII were first detected approximately 24 min after addition of ribonucleoside triphosphates. In contrast, full-length runoff transcripts synthesized in the presence of SIII were detected within 4 min after addition of ribonucleoside triphosphates.

Transcriptional stimulation by SIII is not limited to cases in which one or more of the ribonucleoside triphosphates are present in reaction mixtures at very low concentrations. Analysis of the effect of SIII on transcription at very early times after initiation indicates that the factor also increases the rate of accumulation of full-length runoff transcripts synthesized in the presence of ribonucleoside triphosphates at the concentrations (50 µM ATP, 50 µM UTP, 50 µM GTP, and 10 µM CTP) normally used in our standard transcription assays. Under these conditions, full-length runoff transcripts synthesized in the absence of SIII were first detected ~6 min after addition of ribonucleoside triphosphates. In contrast, full-length runoff transcripts synthesized in the presence of SIII could be detected within 1 min after addition of ribonucleoside triphosphates. Thus, SIII stimulates the rate of RNA chain elongation at least five-fold when transcription is carried out both at very low ribonucleoside triphosphate concentrations and at ribonucleoside triphosphate concentrations used in our standard runoff transcription assays. In addition, we observed that SIII is able to stimulate the rate of RNA chain elongation to a similar extent when all nucleotides are at a concentration of 500 µM.

Although there is synthesis of multiple short transcripts when transcription is carried out in the presence of limiting AMP-PNP or UTP, most of these transcripts can be chased into full-length runoff transcripts either by increasing the concentration of the limiting nucleotide or by addition of SIII indicating that RNA polymerase II pauses at multiple sites during synthesis of transcripts initiated from the AdML promoter on pDN-AdML, (Conaway, R.C. and Conaway, J.W., *J Biol Chem* 263: 2962-2968 (1988)). To determine whether RNA polymerase II pauses at these sites simply as a consequence of limiting levels of ribonucleoside triphosphates or whether polymerase pauses in response to specific DNA sequences that promote pausing regardless of ribonucleoside triphosphate concentration, we compared the lengths of short transcripts synthesized in the presence of low concentrations of each of the four ribonucleoside triphosphates. Distinct sets of RNA products are synthesized in the presence of low concentrations of UTP, ATP, GTP, and CTP, suggesting that most of the RNA polymerase II elongation complexes are not pausing at DNA-sequences that function as specific pause signals. Instead, pause sites appear to be specific for and induced by the limiting ribonucleoside triphosphate. It is noteworthy that, although the 3' termini of RNA in paused elongation complexes have not been established unequivocally, most of them map roughly to DNA sequences encoding two or more sequential residues of the limiting nucleotide (data not shown).

Like SIII, the general initiation factor βγ(TFIIF) has been shown previously to stimulate the rate of RNA chain elongation by RNA polymerase II (price, et al., *Mol Cell Biol* 9: 1465-1475 (1989); Flores, et al., *J Biol Chem* 264: 8913-8921 (1989); Bengal, et al., *Mol Cell Biol* 11: 1195-1206 (1991); Kato, et al., *Genes Dev* 6: 655-666 (1992); and Izban, M.G. and Luse, D.S., *J Biol Chem* 267: 13647-13655 (1992)). The ability of the two factors to stimulate transcription initiated at the oligo-dC tail of pCpGR220 S/P/X or at the AdML promoter was compared. The recombinant βγ(TFIIF) used in these experiments was expressed in and purified from insect cells and was more than 95% pure.

In another procedure, transcription was initiated by addition of purified RNA polymerase II to reaction mixtures containing pCpGR220 S/P/X and 50 µM ATP, 50 µM GTP, and 2 µM [$\alpha^{32}$-P CTP. After a 30 min incubation to allow synthesis of short, labelled transcripts, the accumulated transcripts were chased with 500 µM non-radioactive CTP, ATP, GTP, and UTP, in the presence or absence of SIII or βγ(TFIIF). Under these conditions, both βγ(TFIIF) and SIII strongly stimulate the rate of RNA chain elongation. We estimate that transcription elongation in the presence of either factor is approximately 500 nucleotides/minute.

Preinitiation complexes were assembled at the AdML promoter by preincubation of template DNA with RNA polymerase II and initiation factors; reactions contained an amount of βγ(TFIIF) just sufficient to produce maximal levels of initiation. Transcription was then initiated by addition of ATP, GTP, and a low concentration of UTP and [$\alpha^{32}$-P]CTP. After a 12 min incubation to allow synthesis of short labelled transcripts, the radioactive CTP was diluted by addition of an approximately 30-fold excess of non-radioactive CTP, and varying amounts of SIII or additional $\beta\gamma$(TFIIF) were added to reactions. Under some of these conditions, SIII strongly stimulates accumulation of full length runoff transcripts from the AdML promoter. Likewise, addition of $\beta\gamma$(TFIIF), in quantities greater than that sufficient to saturate the reaction for initiation, stimulates accumulation of full length runoff transcripts.

## Claims

1. A substantially purified SIII protein substantially free of contaminants and other proteins.

2. The SIII protein of claim 1 wherein said SIII protein has a molecular weight of about 140 kilodaltons.

3. The SIII protein of claim 2 wherein said SIII protein is a heterotrimer comprising a single copy each of: a) a first subunit having a molecular weight of about 110 kilodaltons; b) a second subunit having a molecular weight of about 18 kilodaltons; and c) a third subunit having a molecular weight of about 15 kilodaltons.

4. The SIII protein of claim 3 wherein each of said first, second and third subunits move as a distinct single peak on reverse-phase high-performance liquid chromatography when denatured prior to loading.

5. The SIII protein of claim 4 wherein the approximately 15, 18 and 110 kilodalton subunits comprise a primary structure corresponding to the amino acid sequence in SEQ ID NO:2, SEQ ID NO:4, and SEQ ID NO:6, respectively.

6. The SIII protein of claim 4 wherein the approximately 15 and 18 kilodalton subunits comprise a primary structure corresponding to the amino acid sequence in SEQ ID NO:8 and SEQ ID NO:10, respectively.

7. A substantially purified subunit of the SIII protein substantially free of contaminants and other proteins.

8. The purified subunit of claim 7 wherein said subunit has a molecular weight of about 15 kilodaltons.

**9.** The subunit of claim 8 wherein said subunit comprises a primary structure corresponding to the amino acid sequence in SEQ ID NO:2.

**10.** The subunit of claim 8 wherein said subunit comprises a primary structure corresponding to the amino acid sequence in SEQ ID NO:8.

**11.** The purified subunit of claim 7 wherein said subunit has a molecular weight of about 18 kilodaltons.

**12.** The subunit of claim 11 wherein said subunit comprises a primary structure corresponding to the amino acid sequence in SEQ ID NO:4.

**13.** The subunit of claim 11 wherein said subunit comprises a primary structure corresponding to the amino acid sequence in SEQ ID NO:10.

**14.** The purified subunit of claim 7 wherein said subunit has a molecular weight of about 110 kilodaltons.

**15.** The subunit of claim 14 wherein said subunit comprises a primary structure corresponding to the amino acid sequence in SEQ ID NO:6.

**16.** A process for purifying SIII enzyme which comprises:
a. isolating proteins from eukaryotic cells; and
b. fractionating, concentrating and purifying the SIII activity.

**17.** The process of claim 16 wherein said eukaryotic cells are liver cells.

**18.** The process of claim 16 wherein said eukaryotic cells are brain cells.

**19.** The process of claim 16 wherein said eukaryotic cells are peripheral blood lymphocytes.

**20.** The process of claim 16 wherein said eukaryotic cells are umbilical vein endothelial cells.

**21.** A substantially purified SIII enzyme produced in accordance with the process of claim 16.

**22.** A nucleic acid fragment comprising a nucleotide sequence encoding an approximately 15 kilodalton subunit of the SIII protein.

**23.** The nucleic acid fragment of claim 22 wherein the nucleotide sequence encoding the approximately 15 kilodalton subunit of the SIII protein comprises nucleotides 46 to 381 in SEQ ID NO:1, or any nucleic acid fragment substantially homologous therewith.

**24.** The nucleic acid fragment of claim 22 wherein the nucleotide sequence encoding the approximately 15 kilodalton subunit of the SIII protein comprises nucleotides 88 to 426 in SEQ ID NO:7, or any nucleic acid fragment substantially homologous therewith.

**25.** A nucleic acid fragment comprising a nucleotide sequence encoding an approximately 18 kilodalton subunit of the SIII protein.

**26.** The nucleic acid fragment of claim 25 wherein the nucleotide sequence encoding the approximately 18 kilodalton subunit of the SIII protein comprises nucleotides 1 to 354 in SEQ ID NO:3, or any nucleic acid fragment substantially homologous therewith.

**27.** The nucleic acid fragment of claim 25 wherein the nucleotide sequence encoding the approximately 18 kilodalton subunit of the SIII protein comprises nucleotides 2 to 316 in SEQ ID NO:9, or any nucleic acid fragment substantially homologous therewith.

**28.** A nucleic acid fragment comprising a nucleotide sequence encoding an approximately 110 kilodalton subunit of the SIII protein.

**29.** The nucleic acid fragment of claim 28 wherein the nucleotide sequence encoding the approximately 110 kilodalton subunit of the SIII protein comprises nucleotides 82 to 2403 in SEQ ID NO:5, or any nucleic acid fragment substantially homologous therewith.

**30.** The nucleic acid fragment of claim 28 wherein the nucleotide sequence encoding the approximately 110 kilodalton subunit of the SIII protein comprises nucleotides 1 to 727 in SEQ ID NO:11, or any nucleic acid fragment substantially homologous therewith.

**31.** A process of modulating the transcription rate of RNA polymerase II comprising varying the concentration of SIII protein available to RNA polymerase II during transcription.

**32.** The process of claim 31 wherein said modulation occurs in vitro.

**33.** A process which comprises expressing a DNA sequence encoding a SIII transcription factor in a recombinant host transformed with an expression vector containing said DNA sequence.

**34.** The process according to claim 33 wherein said DNA is in the antisense orientation.

**35.** A process which comprises expressing a DNA sequence encoding a subunit of a SIII transcription factor in a recombinant host transformed with an expression vector containing said DNA sequence.

**36.** The process according to claim 35 wherein said DNA is in the antisense orientation.

**37.** A recombinant DNA molecule capable of functioning as a vector comprising a nucleotide sequence encoding an approximately 15 kilodalton subunit of the SIII protein.

**38.** The recombinant DNA molecule of claim 35 wherein the nucleotide sequence encoding the approximately 15 kilodalton subunit of the SIII protein comprises nucleotides 46 to 381 in SEQ ID NO:1, or any nucleic acid fragment substantially homologous therewith.

**39.** The recombinant DNA molecule of claim 35 wherein the nucleotide sequence encoding the approximately 15 kilodalton subunit of the SIII protein comprises nucleotides 88 to 426 in SEQ ID NO:7, or any nucleic acid fragment substantially homologous therewith.

**40.** A recombinant DNA molecule capable of functioning as a vector comprising a nucleotide sequence encoding an approximately 18 kilodalton subunit of the SIII protein.

**41.** The recombinant DNA molecule of claim 38 wherein the nucleotide sequence encoding the approximately 18 kilodalton subunit of the SIII protein comprises nucleotides 1 to 354 in SEQ ID NO:3, or any nucleic acid fragment substantially homologous therewith.

**42.** The recombinant DNA molecule of claim 38 wherein the nucleotide sequence encoding the approximately 18 kilodalton subunit of the SIII protein comprises nucleotides 2 to 316 in SEQ ID NO:9, or any nucleic acid fragment substantially homologous therewith.

**43.** A recombinant DNA molecule capable of functioning as a vector comprising a nucleotide sequence encoding an approximately 110 kilodalton subunit of the SIII protein.

**44.** The recombinant DNA molecule of claim 41 wherein the nucleotide sequence encoding the approximately 110 kilodalton subunit of the SIII protein comprises nucleotides 82 to 2403 in SEQ ID NO:5, or any nucleic acid fragment substantially homologous therewith.

**45.** The recombinant DNA molecule of claim 41 wherein the nucleotide sequence encoding the approximately 110 kilodalton subunit of the SIII protein comprises nucleotides 1 to 727 in SEQ ID NO:11, or any nucleic acid fragment substantially homologous therewith.

**46.** A probe corresponding to less than the entire DNA sequence encoding a subunit of a SIII transcription factor.

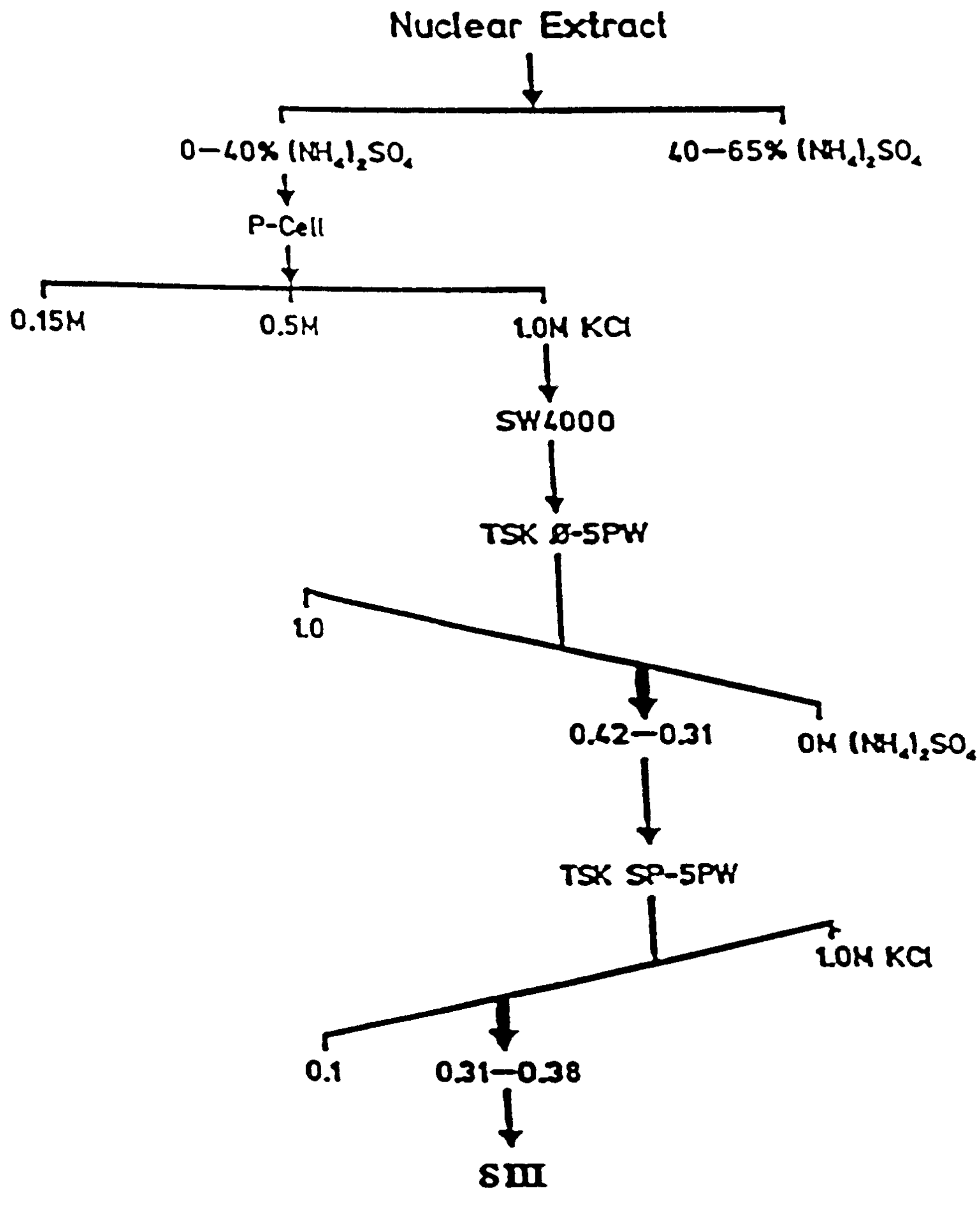
Nuclear Extract
0–40% $(NH_4)_2SO_4$
40–65% $(NH_4)_2SO_4$
P-Cell
0.15M
0.5M
1.0M KCl
SW4000
TSK Ø-5PW
1.0
0.42—0.31
0M $(NH_4)_2SO_4$
TSK SP-5PW
1.0M KCl
0.1
0.31—0.38
SIII

Fig. 1

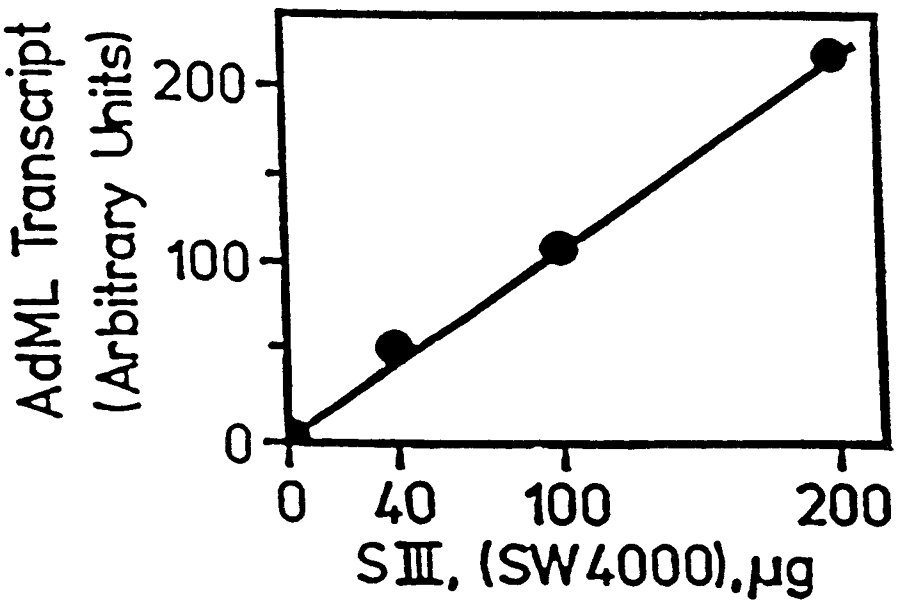
AdML Transcript
(Arbitrary Units)
200
100
0
0
40
100
200
SIII, (SW4000),μg

Fig. 2

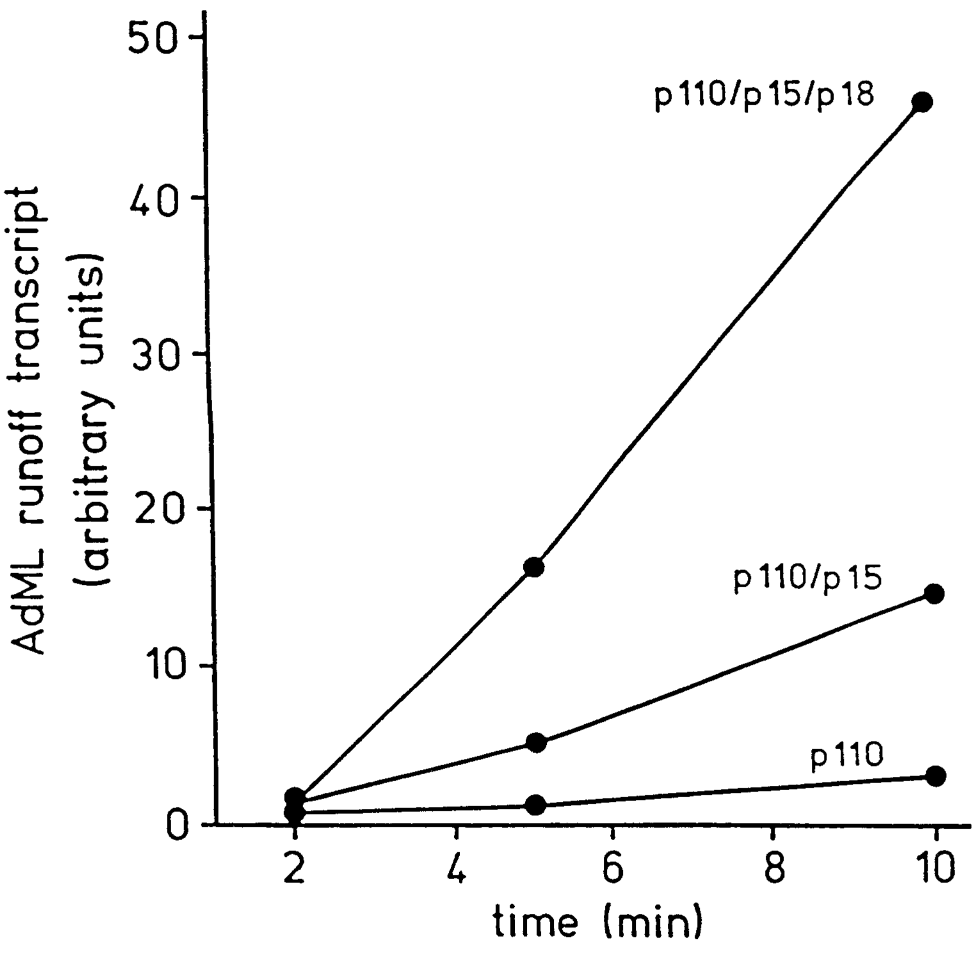
50
40
30
20
10
0
AdML runoff transcript
(arbitrary units)
p110/p15/p18
p110/p15
p110
2
4
6
8
10
time (min)

Fig. 3

European Patent Office

# EUROPEAN SEARCH REPORT

Application Number
EP 94 25 0286

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | JOURNAL OF BIOLOGICAL CHEMISTRY, vol.266, no.14, 15 May 1991, BALTIMORE, MD US pages 9256 - 9262 DAVID R. CHAFIN ET AL. 'Identification and purification of a yeast protein that affects elongation by RNA polymerase II' * abstract * * page 9257, left column, paragraph 2 * * page 9257, right column, paragraph 2 - page 9258, left column, paragraph 1 * * page 9258, left column, paragraph 2 - right column, paragraph 1 * --- | 1,7,14, 16,21, 31,32 | C12N15/12<br>C12N15/70<br>C07K14/47<br>C07K1/14<br>C12Q1/68 |
| A | JOURNAL OF BIOLOGICAL CHEMISTRY, vol.266, no.31, 5 November 1991, BALTIMORE, MD US pages 20940 - 20945 MATTHIEU GERARD ET AL. 'Purification and interaction properties of the human RNA polymerase B(II) general transcription factor BTF2' * abstract * * page 20941, right column, paragraph 1 - page 20942, left column, paragraph 1 * * page 20944, left column, paragraph 4 - page 20945, left column, paragraph 1 * --- | 1-3,16, 21 | **TECHNICAL FIELDS SEARCHED (Int.Cl.6)**<br>C07K<br>C12N<br>C12Q |
| P,X | JOURNAL OF BIOLOGICAL CHEMISTRY, vol.268, no.34, 5 December 1993, BALTIMORE, MD US pages 25587 - 25593 JOHN N. BRADSHER ET AL. 'RNA polymerase II transcription factor SIII' * abstract * * page 25587, right column, paragraph 2 * * page 25591, left column, paragraph 2 - page 25592, right column, paragraph 2 * --- | 1-4,7,8, 11,14, 16,17,21 | |

-/--

The present search report has been drawn up for all claims

| Place of search | Date of completion of the search | Examiner |
|---|---|---|
| THE HAGUE | 23 February 1995 | Montero Lopez, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number
EP 94 25 0286

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | CELLULAR & MOLECULAR BIOLOGY RESEARCH, vol.39, no.4, 1993 pages 323 - 329 JOAN WELIKY CONAWAY ET AL. 'Transcription factor SIII: a novel component of the RNA polymerase II elongation complex' * abstract * * page 324, right column, paragraph 4 * --- | 1-4,7,8, 11,14, 16,17,21 | |
| P,X | PROCEEDINGS OF THE NATIONAL ACADEMY OF SCIENCES OF USA., vol.91, no.12, 7 June 1994, WASHINGTON US pages 5237 - 5241 KARLA PFEIL GARRET ET AL. 'Molecular cloning of an essential subunit of RNA polymerase II elongation factor III' * abstract * * page 5238, right column, paragraph 2 - page 5239, left column, paragraph 1 * * page 5240, right column, paragraph 2 - page 5241, left column, paragraph 1 * ----- | 1-3, 7-10, 21-24, 35, 37-39,46 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 23 February 1995 | Montero Lopez, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

SEQUENCE LISTING

```
(1) GENERAL INFORMATION:

     (i) APPLICANT: Conaway, Ronald C.
                    Conaway, Joan W.
                    Bradsher, John N.

    (ii) TITLE OF INVENTION: RNA Polymerase Transcription Factor

   (iii) NUMBER OF SEQUENCES: 34

    (iv) CORRESPONDENCE ADDRESS:
          (A) ADDRESSEE: RICHARDS, MEDLOCK & ANDREWS
          (B) STREET: 1201 Elm Street, Suite 4500
          (C) CITY: Dallas
          (D) STATE: TX
          (E) COUNTRY: US
          (F) ZIP: 75270-2197

     (v) COMPUTER READABLE FORM:
          (A) MEDIUM TYPE: Floppy disk
          (B) COMPUTER: IBM PC compatible
          (C) OPERATING SYSTEM: PC-DOS/MS-DOS
          (D) SOFTWARE: PatentIn Release #1.0, Version #1.25

    (vi) CURRENT APPLICATION DATA:
          (A) APPLICATION NUMBER:
          (B) FILING DATE:
          (C) CLASSIFICATION:

   (vii) PRIOR APPLICATION DATA:
          (A) APPLICATION NUMBER: US 08/160087
          (B) FILING DATE: 30-NOV-1993

  (viii) ATTORNEY/AGENT INFORMATION:
          (A) NAME: Hansen, Eugenia S.
          (B) REGISTRATION NUMBER: 31,966
          (C) REFERENCE/DOCKET NUMBER: B35006CIP

    (ix) TELECOMMUNICATION INFORMATION:
          (A) TELEPHONE: (214) 939-4500
          (B) TELEFAX: (214) 939-4600


(2) INFORMATION FOR SEQ ID NO:1:

     (i) SEQUENCE CHARACTERISTICS:
          (A) LENGTH: 458 base pairs
          (B) TYPE: nucleic acid
          (C) STRANDEDNESS: both
          (D) TOPOLOGY: both

    (ii) MOLECULE TYPE: cDNA

    (ix) FEATURE:
          (A) NAME/KEY: CDS
          (B) LOCATION: 46..381
```

(xi) SEQUENCE DESCRIPTION: SEQ ID NO:1:

```
ACTAGTAACT TCCTCTGGGA TCAAATAGAA TTTTATAAGA ACACA ATG GAT GGA         54
                                                  Met Asp Gly
                                                    1

GAG GAG AAA ACC TAT GGT GGC TGT GAA GGC CCT GAT GCC ATG TAT GTG      102
Glu Glu Lys Thr Tyr Gly Gly Cys Glu Gly Pro Asp Ala Met Tyr Val
     5                  10                  15

AAA TTA ATA TCT TCT GAT GGT CAT GAA TTT ATT GTA AAA AGA GAA CAT      150
Lys Leu Ile Ser Ser Asp Gly His Glu Phe Ile Val Lys Arg Glu His
 20                  25                  30                  35

GCA TTA ACA TCA GGA ACA ATA AAG GCC ATG TTG AGT GGT CCA GGT CAG      198
Ala Leu Thr Ser Gly Thr Ile Lys Ala Met Leu Ser Gly Pro Gly Gln
                 40                  45                  50

TTT GCG GAG AAT GAA ACC AAT GAG GTC AAC TTT AGA GAG ATC CCT TCA      246
Phe Ala Glu Asn Glu Thr Asn Glu Val Asn Phe Arg Glu Ile Pro Ser
             55                  60                  65

CAT GTG CTA TCG AAA GTG TGC ATG TAT TTT ACC TAC AAG GTC CGC TAT      294
His Val Leu Ser Lys Val Cys Met Tyr Phe Thr Tyr Lys Val Arg Tyr
         70                  75                  80

ACT AAC AGC TCC ACT GAA ATT CCT GAA TTC CCA ATT GCA CCT GAA ATT      342
Thr Asn Ser Ser Thr Glu Ile Pro Glu Phe Pro Ile Ala Pro Glu Ile
     85                  90                  95

GCA CTG GAA CTG CTG ATG GCC GCG AAC TTC CTA GAT TGT TAAATAAAAT       391
Ala Leu Glu Leu Leu Met Ala Ala Asn Phe Leu Asp Cys
100                 105                 110

AAATTATAAT AAACTGTTAA AAAAAAAAAA AAAAAAAAAA AAAAAAAAAA AAAAAAAAAA    451

AAAAAAA                                                              458
```

(2) INFORMATION FOR SEQ ID NO:2:

(i) SEQUENCE CHARACTERISTICS:
(A) LENGTH: 112 amino acids
(B) TYPE: amino acid
(D) TOPOLOGY: linear

(ii) MOLECULE TYPE: protein

(xi) SEQUENCE DESCRIPTION: SEQ ID NO:2:

```
Met Asp Gly Glu Glu Lys Thr Tyr Gly Gly Cys Glu Gly Pro Asp Ala
  1               5                  10                  15

Met Tyr Val Lys Leu Ile Ser Ser Asp Gly His Glu Phe Ile Val Lys
             20                  25                  30

Arg Glu His Ala Leu Thr Ser Gly Thr Ile Lys Ala Met Leu Ser Gly
        35                  40                  45
```

```
Pro Gly Gln Phe Ala Glu Asn Glu Thr Asn Glu Val Asn Phe Arg Glu
     50                  55                  60

Ile Pro Ser His Val Leu Ser Lys Val Cys Met Tyr Phe Thr Tyr Lys
 65                  70                  75                  80

Val Arg Tyr Thr Asn Ser Ser Thr Glu Ile Pro Glu Phe Pro Ile Ala
                 85                  90                  95

Pro Glu Ile Ala Leu Glu Leu Leu Met Ala Ala Asn Phe Leu Asp Cys
            100                 105                 110
```

(2) INFORMATION FOR SEQ ID NO:3:

(i) SEQUENCE CHARACTERISTICS:
(A) LENGTH: 354 base pairs
(B) TYPE: nucleic acid
(C) STRANDEDNESS: both
(D) TOPOLOGY: both

(ii) MOLECULE TYPE: cDNA

(ix) FEATURE:
(A) NAME/KEY: CDS
(B) LOCATION: 1..354

(xi) SEQUENCE DESCRIPTION: SEQ ID NO:3:

```
ATG GAC GTG TTT CTC ATG ATC CGG CGC CAC AAG ACC ACC ATC TTT ACG          48
Met Asp Val Phe Leu Met Ile Arg Arg His Lys Thr Thr Ile Phe Thr
  1               5                  10                  15

GAC GCC AAG GAG TCG AGC ACG GTG TTC GAA CTG AAG CGC ATC GTC GAG          96
Asp Ala Lys Glu Ser Ser Thr Val Phe Glu Leu Lys Arg Ile Val Glu
             20                  25                  30

GGC ATC CTC AAG CGG CCG CCA GAG GAG CAG CGG CTG TAC AAG GAC GAC         144
Gly Ile Leu Lys Arg Pro Pro Glu Glu Gln Arg Leu Tyr Lys Asp Asp
         35                  40                  45

CAG CTC CTT GAT GAT GGA AAA ACT CTG GGC GAA TGT GGC TTC ACC AGT         192
Gln Leu Leu Asp Asp Gly Lys Thr Leu Gly Glu Cys Gly Phe Thr Ser
     50                  55                  60

CAG ACA GCA AGG CCA CAG GCC CCA GCC ACA GTG GGC CTG GCC TTT CGA         240
Gln Thr Ala Arg Pro Gln Ala Pro Ala Thr Val Gly Leu Ala Phe Arg
 65                  70                  75                  80

GCA GAT GAC ACC TTT GAA GCG CTG CGT ATT GAA CCC TTC TCC AGC CCT         288
Ala Asp Asp Thr Phe Glu Ala Leu Arg Ile Glu Pro Phe Ser Ser Pro
                 85                  90                  95

CCG GAG CTT CCA GAT GTG ATG AAG CCA CAG GAT TCT GGA GGC AGT GCC         336
Pro Glu Leu Pro Asp Val Met Lys Pro Gln Asp Ser Gly Gly Ser Ala
            100                 105                 110

AAT GAA CAA GCT GTG CAG                                                 354
Asn Glu Gln Ala Val Gln
        115
```

(2) INFORMATION FOR SEQ ID NO:4:

(i) SEQUENCE CHARACTERISTICS:
(A) LENGTH: 118 amino acids
(B) TYPE: amino acid
(D) TOPOLOGY: linear

(ii) MOLECULE TYPE: protein

(xi) SEQUENCE DESCRIPTION: SEQ ID NO:4:

```
Met Asp Val Phe Leu Met Ile Arg Arg His Lys Thr Thr Ile Phe Thr
 1               5                  10                  15

Asp Ala Lys Glu Ser Ser Thr Val Phe Glu Leu Lys Arg Ile Val Glu
            20                  25                  30

Gly Ile Leu Lys Arg Pro Pro Glu Glu Gln Arg Leu Tyr Lys Asp Asp
        35                  40                  45

Gln Leu Leu Asp Asp Gly Lys Thr Leu Gly Glu Cys Gly Phe Thr Ser
    50                  55                  60

Gln Thr Ala Arg Pro Gln Ala Pro Ala Thr Val Gly Leu Ala Phe Arg
65                  70                  75                  80

Ala Asp Asp Thr Phe Glu Ala Leu Arg Ile Glu Pro Phe Ser Ser Pro
                85                  90                  95

Pro Glu Leu Pro Asp Val Met Lys Pro Gln Asp Ser Gly Gly Ser Ala
            100                 105                 110

Asn Glu Gln Ala Val Gln
        115
```

(2) INFORMATION FOR SEQ ID NO:5:

(i) SEQUENCE CHARACTERISTICS:
(A) LENGTH: 3501 base pairs
(B) TYPE: nucleic acid
(C) STRANDEDNESS: both
(D) TOPOLOGY: both

(ii) MOLECULE TYPE: cDNA

(ix) FEATURE:
(A) NAME/KEY: CDS
(B) LOCATION: 82..2403

(ix) FEATURE:
(A) NAME/KEY: misc_difference
(B) LOCATION: replace(2990, "")
(D) OTHER INFORMATION: /note= "This base can be either a, c, t, g."

(xi) SEQUENCE DESCRIPTION: SEQ ID NO:5:

```
CGTTGCTGTC GAGGGCGGAG TTGCGGCCCG AGGACGCTAC GCGAGCCCAG TTCCGGCGAG        60

GAGGCCGCGC CAGTGACAGC G ATG GCG GCG GAG TCG GCG CTC CAA GTT GTG         111
                        Met Ala Ala Glu Ser Ala Leu Gln Val Val
                          1               5                  10

GAG AAG CTG CAG GCG CGC CTG GCT GCG AAC CCG GAC CCC AAG AAG CTA         159
Glu Lys Leu Gln Ala Arg Leu Ala Ala Asn Pro Asp Pro Lys Lys Leu
                 15                  20                  25

TTG AAA TAT TTG AAG AAA CTT TCC GTC TTA CCT ATT ACA GTA GAC ATT         207
Leu Lys Tyr Leu Lys Lys Leu Ser Val Leu Pro Ile Thr Val Asp Ile
             30                  35                  40

CTT GTG GAG ACT GGG GTG GGG AAA ACA GTG AAC AGC TTT CGG AAA CAT         255
Leu Val Glu Thr Gly Val Gly Lys Thr Val Asn Ser Phe Arg Lys His
         45                  50                  55

GAG CAA GTG GGA AAC TTT GCC AGA GAC CTG GTT GCC CAG TGG AAG AAG         303
Glu Gln Val Gly Asn Phe Ala Arg Asp Leu Val Ala Gln Trp Lys Lys
     60                  65                  70

CTG GTT CCA GTA GAA CGA AAC AAT GAG GCT GAG GAT CAG GAT TTT GAG         351
Leu Val Pro Val Glu Arg Asn Asn Glu Ala Glu Asp Gln Asp Phe Glu
 75                  80                  85                  90

AAG AGC AAT TCC CGC AAG CGT CCC CGA GAT GTT CCC CAG CAG GAG GAG         399
Lys Ser Asn Ser Arg Lys Arg Pro Arg Asp Val Pro Gln Gln Glu Glu
                 95                 100                 105

GAA GCG GAG GGG AAC TAC CAG GAA AGC TGG CAA GCC TCA GGC AGC CAG         447
Glu Ala Glu Gly Asn Tyr Gln Glu Ser Trp Gln Ala Ser Gly Ser Gln
            110                 115                 120

CCC TAC AGC CCT GAG CAC AGA CAG AAA AAG CAC AGA AAA CTT CCT GAG         495
Pro Tyr Ser Pro Glu His Arg Gln Lys Lys His Arg Lys Leu Pro Glu
        125                 130                 135

CTT GAA AGG CCT CAC AAA GTG GCT CAT GGT CAC GAG AGG AGA GAT GAA         543
Leu Glu Arg Pro His Lys Val Ala His Gly His Glu Arg Arg Asp Glu
    140                 145                 150

AGG AAG AGG TGT CAC AAA GTG TCA CCA CCA TAT TCT TCA GAC CCC GAG         591
Arg Lys Arg Cys His Lys Val Ser Pro Pro Tyr Ser Ser Asp Pro Glu
155                 160                 165                 170

TCG TCT GAC TAT GGT CAT GTT CAA TCT CCT CCA CCT TCA AGT CCC CAT         639
Ser Ser Asp Tyr Gly His Val Gln Ser Pro Pro Pro Ser Ser Pro His
                175                 180                 185

CAA ATG TAT ACA GAC CTC TCT AGG TCC CCA GAG ATG GAC CAG GAA CCC         687
Gln Met Tyr Thr Asp Leu Ser Arg Ser Pro Glu Met Asp Gln Glu Pro
            190                 195                 200

ATC GTC TCA CAC CCG AAG CCT GGG AAA GTC CAC AGT AAT ACC TTT CAG         735
Ile Val Ser His Pro Lys Pro Gly Lys Val His Ser Asn Thr Phe Gln
        205                 210                 215
```

```
GAC AGA CTA GGG GTT AGT CAC CTG GGT GAG CAC CAA GGG AAA GGG GCT      783
Asp Arg Leu Gly Val Ser His Leu Gly Glu His Gln Gly Lys Gly Ala
    220                 225                 230

GTT AGC CAA AAC AAG CCA CAC AAA TCT TCC CAC AAG GAG AAA CGC CCT      831
Val Ser Gln Asn Lys Pro His Lys Ser Ser His Lys Glu Lys Arg Pro
235                 240                 245                 250

GTG GAT GCC AGG GGG GAT GAG AAG AGC TCT GTC ATG GGC AGA GAG AAG      879
Val Asp Ala Arg Gly Asp Glu Lys Ser Ser Val Met Gly Arg Glu Lys
                255                 260                 265

TCA CAC AAA GCC TCT TCC AAA GAG GAG AGC CGA AGG CTA CTC TCA GAG      927
Ser His Lys Ala Ser Ser Lys Glu Glu Ser Arg Arg Leu Leu Ser Glu
            270                 275                 280

GAC AGT GCC AAG GAG AAG CTG CCC TCC AGT GTT GTC AAG AAA GAG AAG      975
Asp Ser Ala Lys Glu Lys Leu Pro Ser Ser Val Val Lys Lys Glu Lys
        285                 290                 295

GAC AGA GAA GGC AAC AGC CTC AAG AAG AAG TTG TCA CCT GCC TTA GAT     1023
Asp Arg Glu Gly Asn Ser Leu Lys Lys Lys Leu Ser Pro Ala Leu Asp
    300                 305                 310

GTT GCT TCA GAC AAC CAC TTT AAA AAG CCC AAA CAC AAG GAC TCC GAG     1071
Val Ala Ser Asp Asn His Phe Lys Lys Pro Lys His Lys Asp Ser Glu
315                 320                 325                 330

AAA ATC AAG TCT GAC AAA AAC AAG CAG AGT GTA GAT AGC GTG GAC TCA     1119
Lys Ile Lys Ser Asp Lys Asn Lys Gln Ser Val Asp Ser Val Asp Ser
                335                 340                 345

GGA CGA GGG ACA GGA GAC CCA TTA CCC AGA GCC AAG GAT AAA GTT CCC     1167
Gly Arg Gly Thr Gly Asp Pro Leu Pro Arg Ala Lys Asp Lys Val Pro
            350                 355                 360

AAC AAC CTG AAG GCT CAG GAG GGG AAA GTA AGA ACT AAC TCG GAT CGA     1215
Asn Asn Leu Lys Ala Gln Glu Gly Lys Val Arg Thr Asn Ser Asp Arg
        365                 370                 375

AAG TCA CCA GGC TCA CTC CCT AAA GTA GAA GAG ATG GAC ATG GAT GAT     1263
Lys Ser Pro Gly Ser Leu Pro Lys Val Glu Glu Met Asp Met Asp Asp
    380                 385                 390

GAG TTT GAG CAG CCC ACC ATG TCC TTT GAG TCA TAC CTC AGC TAT GAC     1311
Glu Phe Glu Gln Pro Thr Met Ser Phe Glu Ser Tyr Leu Ser Tyr Asp
395                 400                 405                 410

CAG CCC CGC AAG AAA AAG AAG AAG GTT GTG AAA ACT TCC GGT ACA GCA     1359
Gln Pro Arg Lys Lys Lys Lys Lys Val Val Lys Thr Ser Gly Thr Ala
                415                 420                 425

CTT GGA GAA AAA GGA CTT AAA AAG AAG GAC TCT AAA AGC ACT AGT AAA     1407
Leu Gly Glu Lys Gly Leu Lys Lys Lys Asp Ser Lys Ser Thr Ser Lys
            430                 435                 440

AAC TTG AAC TCG GCT CAG AAA TTA CCC AAG GCG AAT GAA AAC AAG TCA     1455
Asn Leu Asn Ser Ala Gln Lys Leu Pro Lys Ala Asn Glu Asn Lys Ser
        445                 450                 455
```

```
GAC AAG TTG CAG CCA GCT GGA GCC GAA CCC ACG AGG CCT AGA AAG GTC        1503
Asp Lys Leu Gln Pro Ala Gly Ala Glu Pro Thr Arg Pro Arg Lys Val
    460                 465                     470

CCT ACT GAT GTG CTG CCA GCA TTG CCA GAC ATC CCC TTG CCC GCC ATA        1551
Pro Thr Asp Val Leu Pro Ala Leu Pro Asp Ile Pro Leu Pro Ala Ile
475                 480                     485                 490

CAA ACC AAC TAT CGT CCC CTT CCC TCC CTC GAG TTG ATC TCC TCC TTT        1599
Gln Thr Asn Tyr Arg Pro Leu Pro Ser Leu Glu Leu Ile Ser Ser Phe
                495                     500                 505

CAG CCA AAG CGA AAA GCT TTC TCT TCA CCC CAG GAA GAA GAA GAA GCT        1647
Gln Pro Lys Arg Lys Ala Phe Ser Ser Pro Gln Glu Glu Glu Glu Ala
            510                 515                     520

GGG TTC ACA GGA CGC AGA ATG AAT TCT AAG ATG CAG GTG TAT TCA GGT        1695
Gly Phe Thr Gly Arg Arg Met Asn Ser Lys Met Gln Val Tyr Ser Gly
        525                     530                 535

TCC AAG TGT GCC TAT CTC CCC AAA ATG ATG ACC TTG CAC CAG CAG TGT        1743
Ser Lys Cys Ala Tyr Leu Pro Lys Met Met Thr Leu His Gln Gln Cys
    540                 545                     550

ATC CGG GTG CTT AAG AAT AAT ATT GAC TCC ATC TTT GAA GTG GGA GGA        1791
Ile Arg Val Leu Lys Asn Asn Ile Asp Ser Ile Phe Glu Val Gly Gly
555                 560                     565                 570

GTC CCC TAT TCT GTT CTT GAA CCT GTC TTG GAG AGG TGC ACA CCC GAT        1839
Val Pro Tyr Ser Val Leu Glu Pro Val Leu Glu Arg Cys Thr Pro Asp
                575                     580                 585

CAG CTC TAC CGA ATA GAG GAA TGC AAT CAT GTA TTA ATT GAG GAA ACA        1887
Gln Leu Tyr Arg Ile Glu Glu Cys Asn His Val Leu Ile Glu Glu Thr
            590                 595                     600

GAT CAG TTG TGG AAA GTT CAC TGT CAC CGG GAC TTT AAG GAA GAA AGA        1935
Asp Gln Leu Trp Lys Val His Cys His Arg Asp Phe Lys Glu Glu Arg
        605                     610                 615

CCA GAA GAG TAT GAG TCT TGG AGG GAG ATG TAC CTG AGG CTT CAG GAC        1983
Pro Glu Glu Tyr Glu Ser Trp Arg Glu Met Tyr Leu Arg Leu Gln Asp
    620                 625                     630

GCC CGA GAG CAG CGG CTG CGC CTG CTC ACA AAC AAC ATC CGG TCT GCA        2031
Ala Arg Glu Gln Arg Leu Arg Leu Leu Thr Asn Asn Ile Arg Ser Ala
635                 640                     645                 650

CAT GCC AAT AAG CCA AAA GGA CGA CAA GCA AAG ATG GCC TTT GTG AAC        2079
His Ala Asn Lys Pro Lys Gly Arg Gln Ala Lys Met Ala Phe Val Asn
                655                     660                 665

TCT GTG GCC AAG CCA CCG AGA GAT GTT CGA CGG AGG CAG GAG AAG TTT        2127
Ser Val Ala Lys Pro Pro Arg Asp Val Arg Arg Arg Gln Glu Lys Phe
            670                 675                     680

GGA ACC GGG GGA GCA GCT GTC CCT GAG AAA GTC AGG ATT AAG CCA GCA        2175
Gly Thr Gly Gly Ala Ala Val Pro Glu Lys Val Arg Ile Lys Pro Ala
        685                     690                 695
```

```
CCA TAT ACA ACA GGA AGC AGC CAT GTT CCT GCC AGC AAC AGC AGC AGC      2223
Pro Tyr Thr Thr Gly Ser Ser His Val Pro Ala Ser Asn Ser Ser Ser
    700                 705                 710

AGC TTC CAC TCA AGT CCT GAG GAG CTG GCC TAC GAA GGG CCC AGT ACC      2271
Ser Phe His Ser Ser Pro Glu Glu Leu Ala Tyr Glu Gly Pro Ser Thr
715                 720                 725                 730

AGC AGT GCC CAC TTA GCT CCT GTG GCC AGC AGC TCT GTT TCC TAT GAT      2319
Ser Ser Ala His Leu Ala Pro Val Ala Ser Ser Ser Val Ser Tyr Asp
                735                 740                 745

CCC AGG AAA CCA GCT GTG AAG AAA ATT GCC CCG ATG ATG GCC AAG ACC      2367
Pro Arg Lys Pro Ala Val Lys Lys Ile Ala Pro Met Met Ala Lys Thr
            750                 755                 760

ATT AAA GCA TTC AAG AAC AGA TTT TCC CGA CGA TAAACAGGAC TTGCCTTGGA    2420
Ile Lys Ala Phe Lys Asn Arg Phe Ser Arg Arg
        765                 770

GGTTGAGTCT GGAAGCAGGA CTACAGGGAC TATGGGGACG GGAGAAGAGG ATGTCCACAG    2480

AAGACCCGTA TCTTTTGCTC TGTGGAACTT TTGGCCTCTG ACTCCTGCAC AGTTCCAGGT    2540

GTCCCCCTCT GTGCCTCAGC CCTGCACCCT GCTTGTAGCA CACTAGTTTT AATTAATAAA    2600

TATTGCCCCC AGATGTATTT ATAATACCCC AGGGGTTTTT ATTTTATTTT ATTTTCTCTT    2660

TGGTCCACTA TATTTAGCCT TCAGATTTCT GAAGACAGTA TGAGCCTCAG TCTTACTGAG    2720

GACTTTAAGA TCAGTTATAC TTTTGTTGCT AATTAGCATC TTTGTAAACT ATAAGACACT    2780

TTACTTTCCA GGTCCTTTAT TAAAGTTAGA TGAGCCTAGC GAAAGCCATT CTTGTCCTAG    2840

TCAGCTCTTC TGAGTAGACA CTGGTCCTTC AGAGCCAGTG TGTCACTCCA AACCCAGATG    2900

CAGGGCTAGG AGTGAGTGAG TGCCAGGATG CCTGTCCTCC AGGGGAGCCT CCCTGCTGAA    2960

ACCCCCACTG TTCCTTGGTG GGCACTTTTA CTTCAGTAGA TTTGTTAAGG AAGGCCGTTT    3020

GCATCATCTT CAAAGCATAC TACTCTCCCC CATTTTCCAG CTCACTGGAG GACAGAGCCT    3080

AAGAGATGAA CTGATGTAGG ACCTCAGCAG TTTTATAATT TTAGTTTCTG GTTCAGTATT    3140

TTACACACAG ACAGACACAC AGACACACAG ACCCTTCCTT ATTTTGTCTC TCCCTGCACT    3200

GTGGGGAAAC ACACATGACA GGGCTTTTGT AGCTCTGATG TGGTTGGGGG GTTTGAAAAG    3260

CCAGCACTTT ATTTCCTTGT TCTCTATGAA TTCCTACCAG GAGTGAGTGG TTTAGAGCAT    3320

TGTAGTGCTG TGAAGCTTGC TCGTGTTGGA CAGGAAAGGT GCTGACGTGG CCAGAGGAGC    3380

AGCAGTGCAC TGTAGCCAGC AGAGAACTCT GTGAAGCCTC CGCCTTCTGA GTGTTGGCCA    3440

GAGTTAGGGA TGGGCCACCT GCCGAAGGGT AGATGGTCAC CCTGGTCCCA GCCAGTCAGT    3500

T                                                                    3501
```

(2) INFORMATION FOR SEQ ID NO:6:

(i) SEQUENCE CHARACTERISTICS:
(A) LENGTH: 773 amino acids
(B) TYPE: amino acid
(D) TOPOLOGY: linear

(ii) MOLECULE TYPE: protein

(xi) SEQUENCE DESCRIPTION: SEQ ID NO:6:

```
Met Ala Ala Glu Ser Ala Leu Gln Val Val Glu Lys Leu Gln Ala Arg
 1               5                  10                  15

Leu Ala Ala Asn Pro Asp Pro Lys Lys Leu Leu Lys Tyr Leu Lys Lys
            20                  25                  30

Leu Ser Val Leu Pro Ile Thr Val Asp Ile Leu Val Glu Thr Gly Val
        35                  40                  45

Gly Lys Thr Val Asn Ser Phe Arg Lys His Glu Gln Val Gly Asn Phe
    50                  55                  60

Ala Arg Asp Leu Val Ala Gln Trp Lys Lys Leu Val Pro Val Glu Arg
 65                 70                  75                  80

Asn Asn Glu Ala Glu Asp Gln Asp Phe Glu Lys Ser Asn Ser Arg Lys
                85                  90                  95

Arg Pro Arg Asp Val Pro Gln Gln Glu Glu Glu Ala Glu Gly Asn Tyr
            100                 105                 110

Gln Glu Ser Trp Gln Ala Ser Gly Ser Gln Pro Tyr Ser Pro Glu His
        115                 120                 125

Arg Gln Lys Lys His Arg Lys Leu Pro Glu Leu Glu Arg Pro His Lys
    130                 135                 140

Val Ala His Gly His Glu Arg Arg Asp Glu Arg Lys Arg Cys His Lys
145                 150                 155                 160

Val Ser Pro Pro Tyr Ser Ser Asp Pro Glu Ser Ser Asp Tyr Gly His
                165                 170                 175

Val Gln Ser Pro Pro Pro Ser Ser Pro His Gln Met Tyr Thr Asp Leu
            180                 185                 190

Ser Arg Ser Pro Glu Met Asp Gln Glu Pro Ile Val Ser His Pro Lys
        195                 200                 205

Pro Gly Lys Val His Ser Asn Thr Phe Gln Asp Arg Leu Gly Val Ser
    210                 215                 220

His Leu Gly Glu His Gln Gly Lys Gly Ala Val Ser Gln Asn Lys Pro
225                 230                 235                 240

His Lys Ser Ser His Lys Glu Lys Arg Pro Val Asp Ala Arg Gly Asp
                245                 250                 255
```

Glu Lys Ser Ser Val Met Gly Arg Glu Lys Ser His Lys Ala Ser Ser
260 265 270

Lys Glu Glu Ser Arg Arg Leu Leu Ser Glu Asp Ser Ala Lys Glu Lys
275 280 285

Leu Pro Ser Ser Val Val Lys Lys Glu Lys Asp Arg Glu Gly Asn Ser
290 295 300

Leu Lys Lys Lys Leu Ser Pro Ala Leu Asp Val Ala Ser Asp Asn His
305 310 315 320

Phe Lys Lys Pro Lys His Lys Asp Ser Glu Lys Ile Lys Ser Asp Lys
325 330 335

Asn Lys Gln Ser Val Asp Ser Val Asp Ser Gly Arg Gly Thr Gly Asp
340 345 350

Pro Leu Pro Arg Ala Lys Asp Lys Val Pro Asn Asn Leu Lys Ala Gln
355 360 365

Glu Gly Lys Val Arg Thr Asn Ser Asp Arg Lys Ser Pro Gly Ser Leu
370 375 380

Pro Lys Val Glu Glu Met Asp Met Asp Asp Glu Phe Glu Gln Pro Thr
385 390 395 400

Met Ser Phe Glu Ser Tyr Leu Ser Tyr Asp Gln Pro Arg Lys Lys Lys
405 410 415

Lys Lys Val Val Lys Thr Ser Gly Thr Ala Leu Gly Glu Lys Gly Leu
420 425 430

Lys Lys Lys Asp Ser Lys Ser Thr Ser Lys Asn Leu Asn Ser Ala Gln
435 440 445

Lys Leu Pro Lys Ala Asn Glu Asn Lys Ser Asp Lys Leu Gln Pro Ala
450 455 460

Gly Ala Glu Pro Thr Arg Pro Arg Lys Val Pro Thr Asp Val Leu Pro
465 470 475 480

Ala Leu Pro Asp Ile Pro Leu Pro Ala Ile Gln Thr Asn Tyr Arg Pro
485 490 495

Leu Pro Ser Leu Glu Leu Ile Ser Ser Phe Gln Pro Lys Arg Lys Ala
500 505 510

Phe Ser Ser Pro Gln Glu Glu Glu Glu Ala Gly Phe Thr Gly Arg Arg
515 520 525

Met Asn Ser Lys Met Gln Val Tyr Ser Gly Ser Lys Cys Ala Tyr Leu
530 535 540

Pro Lys Met Met Thr Leu His Gln Gln Cys Ile Arg Val Leu Lys Asn
545 550 555 560

Asn Ile Asp Ser Ile Phe Glu Val Gly Gly Val Pro Tyr Ser Val Leu
565 570 575

```
Glu Pro Val Leu Glu Arg Cys Thr Pro Asp Gln Leu Tyr Arg Ile Glu
            580                 585                 590

Glu Cys Asn His Val Leu Ile Glu Glu Thr Asp Gln Leu Trp Lys Val
        595                 600                 605

His Cys His Arg Asp Phe Lys Glu Glu Arg Pro Glu Glu Tyr Glu Ser
    610                 615                 620

Trp Arg Glu Met Tyr Leu Arg Leu Gln Asp Ala Arg Glu Gln Arg Leu
625                 630                 635                 640

Arg Leu Leu Thr Asn Asn Ile Arg Ser Ala His Ala Asn Lys Pro Lys
                645                 650                 655

Gly Arg Gln Ala Lys Met Ala Phe Val Asn Ser Val Ala Lys Pro Pro
            660                 665                 670

Arg Asp Val Arg Arg Arg Gln Glu Lys Phe Gly Thr Gly Gly Ala Ala
        675                 680                 685

Val Pro Glu Lys Val Arg Ile Lys Pro Ala Pro Tyr Thr Thr Gly Ser
    690                 695                 700

Ser His Val Pro Ala Ser Asn Ser Ser Ser Ser Phe His Ser Ser Pro
705                 710                 715                 720

Glu Glu Leu Ala Tyr Glu Gly Pro Ser Thr Ser Ser Ala His Leu Ala
                725                 730                 735

Pro Val Ala Ser Ser Ser Val Ser Tyr Asp Pro Arg Lys Pro Ala Val
            740                 745                 750

Lys Lys Ile Ala Pro Met Met Ala Lys Thr Ile Lys Ala Phe Lys Asn
        755                 760                 765

Arg Phe Ser Arg Arg
    770
```

(2) INFORMATION FOR SEQ ID NO:7:

(i) SEQUENCE CHARACTERISTICS:
(A) LENGTH: 444 base pairs
(B) TYPE: nucleic acid
(C) STRANDEDNESS: both
(D) TOPOLOGY: both

(ii) MOLECULE TYPE: cDNA to mRNA

(vi) ORIGINAL SOURCE:
(A) ORGANISM: Homo sapiens
(F) TISSUE TYPE: Blood
(G) CELL TYPE: Lymphocyte

(ix) FEATURE:
(A) NAME/KEY: CDS
(B) LOCATION: 88..426

(xi) SEQUENCE DESCRIPTION: SEQ ID NO:7:

```
AGATTTGGCA CGAGTCGGCA CGAGGCGGGA CTGACGAGAA ACTACTAAAG TTCCTGGGGA        60

AGCAAAGTAG AATTTCATAA GAACAAA ATG GAT GGA GAG GAG AAA ACC TAT           111
                              Met Asp Gly Glu Glu Lys Thr Tyr
                               1               5

GGT GGC TGT GAA GGA CCT GAT GCC ATG TAT GTC AAA TTG ATA TCA TCT         159
Gly Gly Cys Glu Gly Pro Asp Ala Met Tyr Val Lys Leu Ile Ser Ser
    10                  15                  20

GAT GGC CAT GAA TTT ATT GTA AAA AGA GAA CAT GCA TTA ACA TCA GGC         207
Asp Gly His Glu Phe Ile Val Lys Arg Glu His Ala Leu Thr Ser Gly
 25                  30                  35                  40

ACG ATA AAA GCC ATG TTG AGT GGC CCA GGT CAG TTT GCT GAG AAC GAA         255
Thr Ile Lys Ala Met Leu Ser Gly Pro Gly Gln Phe Ala Glu Asn Glu
                45                  50                  55

ACC AAT GAG GTC AAT TTT AGA GAG ATA CCT TCA CAT GTG CTA TCG AAA         303
Thr Asn Glu Val Asn Phe Arg Glu Ile Pro Ser His Val Leu Ser Lys
            60                  65                  70

GTA TGC ATG TAT TTT ACG TAC AAG GTT CGC TAC ACT AAC AGC TCC ACC         351
Val Cys Met Tyr Phe Thr Tyr Lys Val Arg Tyr Thr Asn Ser Ser Thr
        75                  80                  85

GAG ATT CCT GAA TTC CCA ATT GCA CCT GAA ATT GCA CTG GAA CTG CTG         399
Glu Ile Pro Glu Phe Pro Ile Ala Pro Glu Ile Ala Leu Glu Leu Leu
    90                  95                 100

ATG GCT GCG AAC TTC TTA GAT TGT TAAATAAAAT AAATTATAAT A                 444
Met Ala Ala Asn Phe Leu Asp Cys
105                 110
```

(2) INFORMATION FOR SEQ ID NO:8:

(i) SEQUENCE CHARACTERISTICS:
(A) LENGTH: 112 amino acids
(B) TYPE: amino acid
(D) TOPOLOGY: linear

(ii) MOLECULE TYPE: protein

(xi) SEQUENCE DESCRIPTION: SEQ ID NO:8:

```
Met Asp Gly Glu Glu Lys Thr Tyr Gly Gly Cys Glu Gly Pro Asp Ala
 1               5                  10                  15

Met Tyr Val Lys Leu Ile Ser Ser Asp Gly His Glu Phe Ile Val Lys
            20                  25                  30

Arg Glu His Ala Leu Thr Ser Gly Thr Ile Lys Ala Met Leu Ser Gly
        35                  40                  45

Pro Gly Gln Phe Ala Glu Asn Glu Thr Asn Glu Val Asn Phe Arg Glu
    50                  55                  60
```

```
Ile Pro Ser His Val Leu Ser Lys Val Cys Met Tyr Phe Thr Tyr Lys
 65                 70                  75                  80

Val Arg Tyr Thr Asn Ser Ser Thr Glu Ile Pro Glu Phe Pro Ile Ala
                85                  90                  95

Pro Glu Ile Ala Leu Glu Leu Leu Met Ala Ala Asn Phe Leu Asp Cys
            100                 105                 110
```

(2) INFORMATION FOR SEQ ID NO:9:

(i) SEQUENCE CHARACTERISTICS:
(A) LENGTH: 317 base pairs
(B) TYPE: nucleic acid
(C) STRANDEDNESS: both
(D) TOPOLOGY: both

(ii) MOLECULE TYPE: cDNA

(vi) ORIGINAL SOURCE:
(A) ORGANISM: Homo sapiens

(ix) FEATURE:
(A) NAME/KEY: CDS
(B) LOCATION: 2..316

(xi) SEQUENCE DESCRIPTION: SEQ ID NO:9:

```
C CGG CGC CAC AAG ACC ACC ATC TTC ACG GAC GCC AAG GAG TCC AGC          46
  Arg Arg His Lys Thr Thr Ile Phe Thr Asp Ala Lys Glu Ser Ser
    1               5                  10                  15

ACG GTG TTC GAA CTG AAG CGC ATC GTC GAG GGC ATC CTC AAG CGG CCT        94
Thr Val Phe Glu Leu Lys Arg Ile Val Glu Gly Ile Leu Lys Arg Pro
                20                  25                  30

CCT GAC GAG CAG CGG CTG TAC AAG GAT GAC CAA CTC TTG GAT GAT GGC       142
Pro Asp Glu Gln Arg Leu Tyr Lys Asp Asp Gln Leu Leu Asp Asp Gly
            35                  40                  45

AAG ACA CTG GGC GAG TGT GGC TTC ACC AGT CAA ACA GCA CGG CCA CAG       190
Lys Thr Leu Gly Glu Cys Gly Phe Thr Ser Gln Thr Ala Arg Pro Gln
        50                  55                  60

GCC CCA GCC ACA GTG GGG CTG GCC TTC CGG GCA GAT GAC ACC TTT GAG       238
Ala Pro Ala Thr Val Gly Leu Ala Phe Arg Ala Asp Asp Thr Phe Glu
    65                  70                  75

GCC CTG TGC ATC GAG CCG TTT TCC AGC CCG CCA GAG CTG CCC GAT GTG       286
Ala Leu Cys Ile Glu Pro Phe Ser Ser Pro Pro Glu Leu Pro Asp Val
 80                 85                  90                  95

ATG AAG CCC CAG GAC TCG GGT TGC AGT GCC A                             317
Met Lys Pro Gln Asp Ser Gly Cys Ser Ala
                100                 105
```

(2) INFORMATION FOR SEQ ID NO:10:

(i) SEQUENCE CHARACTERISTICS:
(A) LENGTH: 105 amino acids
(B) TYPE: amino acid
(D) TOPOLOGY: linear

(ii) MOLECULE TYPE: protein

(xi) SEQUENCE DESCRIPTION: SEQ ID NO:10:

```
Arg Arg His Lys Thr Thr Ile Phe Thr Asp Ala Lys Glu Ser Ser Thr
 1               5                  10                  15

Val Phe Glu Leu Lys Arg Ile Val Glu Gly Ile Leu Lys Arg Pro Pro
            20                  25                  30

Asp Glu Gln Arg Leu Tyr Lys Asp Asp Gln Leu Leu Asp Asp Gly Lys
        35                  40                  45

Thr Leu Gly Glu Cys Gly Phe Thr Ser Gln Thr Ala Arg Pro Gln Ala
    50                  55                  60

Pro Ala Thr Val Gly Leu Ala Phe Arg Ala Asp Asp Thr Phe Glu Ala
65                  70                  75                  80

Leu Cys Ile Glu Pro Phe Ser Ser Pro Pro Glu Leu Pro Asp Val Met
                85                  90                  95

Lys Pro Gln Asp Ser Gly Cys Ser Ala
            100                 105
```

(2) INFORMATION FOR SEQ ID NO:11:

(i) SEQUENCE CHARACTERISTICS:
(A) LENGTH: 727 base pairs
(B) TYPE: nucleic acid
(C) STRANDEDNESS: both
(D) TOPOLOGY: both

(ii) MOLECULE TYPE: cDNA

(vi) ORIGINAL SOURCE:
(A) ORGANISM: Homo sapiens

(xi) SEQUENCE DESCRIPTION: SEQ ID NO:11:

```
CGTGGGGAAG GCCTCTGTGG TGAGCAGAGA GAAATCACAC AAGGCCCTCT CCAAAGAGGA      60

GAACCGAAGG CCACCCTCAG GGACAATGCA AGGGAGAAAC CGCCCTCTAG TGGCGTAAAG     120

AAAGAGAAGG ACAGAGAGGG CAGCAGCTGA AGAAGAAGTG TTTGCCTCCC TCAGAGGCCG     180

CTTCAGACAA CCACCTGAAA AAGCCAAAGC ACAGAGACCC AGAGAAAGCC AAATTGGACA     240

AAAGCAAGCA AGGTCTGGAC AGCTTTGACA CAGGAAAAGG AGCAGGAGAC CTGTTGCCCA     300

AGGTAAAAGA GTAAGGGTTC TAACAACCTA AAGACTCCAG AAGGGAAAGT CAAAACTAAT     360
```

```
TTGGATAGAA AGTCACTGGG CTCCCTCCCT AAAGTTGAGG AGACAGATAT GGAGGATGAA      420
TTCGAGCCAG CCAACCATGT CTTTTGAATC CTACCTCAGC TATGACCAGT CCCCGGAAGA      480
AAAAGAAAAA GATTGTGAAA ACTTCAGCCA CGGCACTTGG AGATAAAGGA CTTAAAAAAA      540
ATGACTCTAA AAGCACTGGT AAAAACTTGG ACTCAGTTCA GAAATTACCC AAGGTGAACA      600
AAACCAAGTC AGAGAAGCCG GCTGGAGCTG ATTTAGCCAA GCTGAGAAAG GTGCCTGATG      660
TGTTGCCAGT GTTCCAGACC TCCCGTTACC GCGATACAGG CCAATTACCG TCCACTGCCT      720
TCCCTCG                                                                727
```

(2) INFORMATION FOR SEQ ID NO:12:

(i) SEQUENCE CHARACTERISTICS:
(A) LENGTH: 13 amino acids
(B) TYPE: amino acid
(D) TOPOLOGY: linear

(ii) MOLECULE TYPE: peptide

(xi) SEQUENCE DESCRIPTION: SEQ ID NO:12:

```
Leu Ile Ser Ser Asp Gly His Glu Phe Ile Val Lys Arg
1               5                   10
```

(2) INFORMATION FOR SEQ ID NO:13:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:13:

```
Ala Met Leu Ser Gly Pro Gly Gln Phe Ala Glu Asn Glu Thr Asn Glu
1               5                   10                  15

Val Asn Phe Arg
            20
```

(2) INFORMATION FOR SEQ ID NO:14:

(i) SEQUENCE CHARACTERISTICS:
(A) LENGTH: 8 amino acids
(B) TYPE: amino acid
(D) TOPOLOGY: linear

(ii) MOLECULE TYPE: peptide

(xi) SEQUENCE DESCRIPTION: SEQ ID NO:14:

```
Val Cys Met Tyr Phe Thr Tyr Lys
1               5
```

(2) INFORMATION FOR SEQ ID NO:15:

(i) SEQUENCE CHARACTERISTICS:
(A) LENGTH: 29 amino acids
(B) TYPE: amino acid
(D) TOPOLOGY: linear

(ii) MOLECULE TYPE: peptide

(xi) SEQUENCE DESCRIPTION: SEQ ID NO:15:

```
Tyr Thr Asn Ser Ser Thr Glu Ile Pro Glu Phe Pro Ile Ala Pro Glu
1               5                   10                  15

Ile Ala Leu Glu Leu Leu Met Ala Ala Asn Phe Leu Asp
            20                  25
```

(2) INFORMATION FOR SEQ ID NO:16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: misc_feature
(B) LOCATION: 1..20
(D) OTHER INFORMATION: /note= "degenerate oligonucleotide"

(xi) SEQUENCE DESCRIPTION: SEQ ID NO:16:

CARTTYGCNG ARAAYGARAC 20

(2) INFORMATION FOR SEQ ID NO:17:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: misc_feature
(B) LOCATION: 1..20
(D) OTHER INFORMATION: /note= "degenerate oligonucleotide"

(xi) SEQUENCE DESCRIPTION: SEQ ID NO:17:

GGNGCDATNG GRAAYTCNGG 20

(2) INFORMATION FOR SEQ ID NO:18:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: misc_feature
(B) LOCATION: 1..20
(D) OTHER INFORMATION: /note= "degenerate oligonucleotide"

(xi) SEQUENCE DESCRIPTION: SEQ ID NO:18:

ACNAAYGARG TNAAYTTYMG 20

(2) INFORMATION FOR SEQ ID NO:19:

(i) SEQUENCE CHARACTERISTICS:
(A) LENGTH: 29 amino acids
(B) TYPE: amino acid
(D) TOPOLOGY: linear

(ii) MOLECULE TYPE: peptide

(xi) SEQUENCE DESCRIPTION: SEQ ID NO:19:

```
Leu Tyr Lys Asp Asp Gln Leu Leu Asp Asp Gly Lys Thr Leu Gly Glu
1               5                   10                  15

Cys Gly Phe Thr Ser Gln Thr Ala Arg Pro Gln Ala Pro
            20                  25
```

(2) INFORMATION FOR SEQ ID NO:20:

(i) SEQUENCE CHARACTERISTICS:
(A) LENGTH: 34 amino acids
(B) TYPE: amino acid
(D) TOPOLOGY: linear

(ii) MOLECULE TYPE: peptide

(xi) SEQUENCE DESCRIPTION: SEQ ID NO:20:

```
Ala Asp Asp Thr Gly Glu Ala Leu Arg Ile Glu Pro Phe Ser Ser Pro
1               5                   10                  15

Pro Glu Leu Pro Asp Val Met Lys Pro Gln Asp Ser Gly Gly Ser Ala
            20                  25                  30
Asn Glu
```

(2) INFORMATION FOR SEQ ID NO:21:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: misc_feature
(B) LOCATION: 1..19
(D) OTHER INFORMATION: /note= "degenerate oligonucleotide"

(xi) SEQUENCE DESCRIPTION: SEQ ID NO:21:

TNTAYAARGA YGAYCARYT 19

(2) INFORMATION FOR SEQ ID NO:22:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: misc_feature
(B) LOCATION: 1..20
(D) OTHER INFORMATION: /note= "degenerate oligonucleotide"

(xi) SEQUENCE DESCRIPTION: SEQ ID NO:22:

TGNGGYTTCA TNACRTCNGG 20

(2) INFORMATION FOR SEQ ID NO:23:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: misc_feature
(B) LOCATION: 1..20
(D) OTHER INFORMATION: /note= "degenerate oligonucleotide"

(xi) SEQUENCE DESCRIPTION: SEQ ID NO:23:

GCNGAYGAYA CNTTYGARGC 20

(2) INFORMATION FOR SEQ ID NO:24:

(i) SEQUENCE CHARACTERISTICS:
(A) LENGTH: 30 amino acids
(B) TYPE: amino acid
(D) TOPOLOGY: linear

(ii) MOLECULE TYPE: peptide

(xi) SEQUENCE DESCRIPTION: SEQ ID NO:24:

```
Asp Val Pro Gln Gln Glu Glu Glu Ala Glu Gly Asn Tyr Gln Glu Ser
1               5                   10                  15

Trp Gln Ala Ser Gly Ser Gln Pro Tyr Tyr Pro Glu His Arg
            20                  25                  30
```

(2) INFORMATION FOR SEQ ID NO:25:

(i) SEQUENCE CHARACTERISTICS:
(A) LENGTH: 19 amino acids
(B) TYPE: amino acid
(D) TOPOLOGY: linear

(ii) MOLECULE TYPE: peptide

(xi) SEQUENCE DESCRIPTION: SEQ ID NO:25:

```
Ala Asn Glu Asn Lys Ser Asp Lys Leu Gln Pro Ala Gly Ala Glu Pro
1               5                   10                  15

Thr Arg Pro
```

(2) INFORMATION FOR SEQ ID NO:26:

(i) SEQUENCE CHARACTERISTICS:
(A) LENGTH: 43 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: modified_base
(B) LOCATION: 12
(D) OTHER INFORMATION: /mod_base= i

(ix) FEATURE:
(A) NAME/KEY: modified_base
(B) LOCATION: 17
(D) OTHER INFORMATION: /mod_base= i

(ix) FEATURE:
(A) NAME/KEY: modified_base
(B) LOCATION: 35
(D) OTHER INFORMATION: /mod_base= i

(ix) FEATURE:
(A) NAME/KEY: modified_base
(B) LOCATION: 38
(D) OTHER INFORMATION: /mod_base= i

(xi) SEQUENCE DESCRIPTION: SEQ ID NO:26:

TCCTGGTAGT TNCCTCNGCC TCCTCCTCCT GCTGNGGNAC GTC 43

(2) INFORMATION FOR SEQ ID NO:27:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: modified_base
(B) LOCATION: 9
(D) OTHER INFORMATION: /mod_base= i

(ix) FEATURE:
(A) NAME/KEY: modified_base
(B) LOCATION: 15
(D) OTHER INFORMATION: /mod_base= i

(ix) FEATURE:
(A) NAME/KEY: modified_base
(B) LOCATION: 18
(D) OTHER INFORMATION: /mod_base= i

(ix) FEATURE:
(A) NAME/KEY: modified_base
(B) LOCATION: 21
(D) OTHER INFORMATION: /mod_base= i

(ix) FEATURE:
(A) NAME/KEY: modified_base
(B) LOCATION: 24
(D) OTHER INFORMATION: /mod_base= i

(xi) SEQUENCE DESCRIPTION: SEQ ID NO:27:

CGGATCGTNG GYTCNGCNCC NGCNGGYTG 29

(2) INFORMATION FOR SEQ ID NO:28:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:28:

CGTAATACGA CTCACTATAG GG 22

(2) INFORMATION FOR SEQ ID NO:29:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:29:

```
Met His His His His His His Asn Val Asp
1               5                   10
```

(2) INFORMATION FOR SEQ ID NO:30:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:30:

```
Met His His His His His His Asn Val Asp
1               5                   10
```

(2) INFORMATION FOR SEQ ID NO:31:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:31:

GCAGCGGATC CTCAACAATC TAGGAAGTTC G 31

(2) INFORMATION FOR SEQ ID NO:32:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:32:

GCAACGTCGA CATGGACGTG TTTCTCATGA T 31

(2) INFORMATION FOR SEQ ID NO:33:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:33:

GCAGCGGATC CTCACTGCAC AGCTTGTTCA T 31

(2) INFORMATION FOR SEQ ID NO:34:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:34:

GCNGAYGAYA CNTTYGARGC 20